# EUROPEAN PATENT SPECIFICATION

(11) **EP 3 769 718 B1**
(45) Date of publication and mention of the grant of the patent: **31.12.2025**
(21) Application number: 20160628.2
(22) Date of filing: 10.10.2008
(51) Int. Cl.: A61F 2/04, A61M 1/00, A61N 1/36, A61N 1/05

(54) **APPARATUS FOR TREATING INTESTINAL DISORDER**
VORRICHTUNG ZUR BEHANDLUNG VON DARMERKRANKUNGEN
APPAREIL DE TRAITEMENT D'UN TROUBLE INTESTINAL

(30) Priority: 12.10.2007 US 960767 P
(43) Date of publication of application: 27.01.2021
(62) Divisional of application: 08837916.9
(73) Proprietor: Implantica Patent Ltd., 223 70 Lund (SE)
(72) Inventor: Forsell, Peter, 6341 Baar (CH)
(74) Representative: AWA Sweden AB

(56) References cited:
- US-A1- 2004 122 526
- US-A1- 2004 122 527

## Description

### FIELD OF THE INVENTION

The present invention relates to an apparatus for treating a patient having a disorder related to the passageway of the patient's intestines, such as constipation or incontinence.

Anal incontinence is a widespread problem. Many different solutions to this problem have been tried, and there are several kinds of sphincter surgery currently in use to remedy anal incontinence. For example, there is a prior manually operable implanted system including a hydraulic artificial anal sphincter and a balloon reservoir connected to the anal sphincter and placed in the patient's scrotum. The disadvantage of this implanted system is that hard fibrosis formed around the reservoir over time may cause the malfunction of the system's pumping components. Thus, the formed fibrosis will sooner or later become a hard fibrotic layer, which may make it difficult to pump the balloon reservoir. Yet a further disadvantage is that the system's use of hydraulic fluid always entails a risk of the fluid leaking from the system. Furthermore, it is a rather complicated task to manually pump the reservoir when defecation is needed.

U.S. Patent No. 5,593,443 discloses an artificial hydraulic anal sphincter under both reflex and voluntary control. An inflatable artificial sphincter with a pump system in the patient's scrotum is disclosed in U.S. Patent No. 4,222,377.

An artificial and special form of anal incontinence is caused by ileostomy, jejunostomy, colostomy and rectostomy operations. Thus, the small intestine (the jejunum or ileum) or the large intestine (the colon or rectum) is cut and the open end portion of the intestine is passed trough the patient's abdominal wall. The opening at the end of the intestine is referred to as the "stoma". The reason for such an operation may be colorectal cancer, perforated diverticulitis or different kinds of diseases in the intestine, such as ulceros colitis or Crohns disease. Typically, a patient having a stoma wears a plastic bag that continuously collects the intestinal contents that is discharged through the stoma. Making such a bag arrangement liquid tight requires an adhesive plate that is attached to the patient's skin. The adhesive plate is inconvenient for the patient and often makes the skin red and irritated. It is also inconvenient for the patient to always wear a bag which contains fecal matter, and which is typically placed on the patient's belly.

U.S. Patent No. 6,752,754 discloses an artificial rectum for replacing a diseased portion of a patient's rectum. An inlet of the artificial rectum is operatively connected to the distal end of the patient's large intestine and communicates fecal matter to a macerator-type pump that discharges the feces through an outlet of the artificial rectum connected to the patient's anus. The pump includes a helical screw-type impeller, which when rotated creates a shearing effect on the feces, causing it to move down the thread of the screw impeller and discharge through the patient's anus. Patent publication US 2004/122527A1 discloses an artificial piece of intestine with two tubes, one inserted into the other.

### SUMMARY OF THE INVENTION

An object of the present invention is to provide a convenient apparatus for treating a patient having a disorder related to the passageway of the patient's intestines.

Another object of the present disclosure is to provide methods for treating a patient having a disorder related to the passageway of the patient's intestines.

Accordingly, in accordance with a first aspect of the present invention, there is provided an apparatus for treating a patient having a disorder related to the passageway of the patient's intestines, comprising an electric pump for implantation in the patient, the electric pump being operable on at least one selected portion of the intestines to pump intestinal contents through the passageway of the intestines, wherein the electric pump includes a constriction device adapted to alternately constrict the selected portion to at least substantially reduce the volume of the passageway of the intestines along the selected portion and release the selected portion to increase the volume of the passageway of the intestines along the selected portion, such that intestinal contents is displaced through the passageway of the intestines.

For a patient where the selected portion of the intestines ends at the patient's anus, the pump is adapted to pump intestinal contents out from the patient's body through the anus. This solution differs from the prior art according to U.S. 6,752,754 discussed above, in that the pump of the present invention operates on the patient's intestine, *i.e.,* the constriction device of the pump constricts the intestine to displace intestinal contents therein.

For an ileostomy, a jejunostomy, a colostomy or a rectostomy patient, where the patient's intestines are surgically modified ending in a stoma, the pump is adapted to pump intestinal contents out from the patient's body through the stoma.

The apparatus of the invention may include an artificial intestinal piece adapted to be surgically joined to the patient's intestines to form part of the passageway of the intestines and to form at least part of the selected portion of the intestines to be constricted by the constriction device. The constriction device may operate only on the artificial intestinal piece to minimize the risk of injuring the intestines. When the pump is not in operation, the constriction device can firmly constrict the artificial intestinal piece to completely close the passageway of the intestines. Alternatively, there may be provided at least one implantable releasable closure adapted to engage the artificial intestinal piece to close the passageway of the intestines, or at least partially constrict the selected portion, when the pump is not in operation, and to release the selected portion to open the passageway of the intestines when the pump is in operation.

The artificial intestinal piece may be integrated with the patient's intestines between two ends thereof. Specifically, the artificial intestinal piece may be joined directly or indirectly to the patient's anus, whereby the pump can pump intestinal contents out from the patient's body through the anus. This solution differs from the prior art according to U.S. 6,752,754 discussed above, in that the pump of the present invention operates on the artificial intestinal piece, i.e., the constriction device of the pump constricts the artificial intestinal piece to displace intestinal contents therein. Alternatively, the artificial intestinal piece may be adapted to end in a stoma, whereby the pump can to pump intestinal contents out from the patient's body through the stoma.

To keep the pump fixed in a desired position in the patient's abdomen, an engagement device may be attached to the pump, the engagement device being adapted to attach the pump to tissue related to the patient's abdominal cavity, such as the abdominal wall.

To externally protect the intestines, an elastic protective tubing may be provided to at least partially cover the selected portion of the intestines, so that the constriction device of the pump constricts both the protective tubing and the selected portion.

An implantable support may be provided to support the selected portion of the intestines as the constriction device constricts the selected portion. Alternatively, the constriction device may be adapted to constrict the selected portion against a tissue or a bone of the patient's body.

The apparatus further comprises a control device for controlling the pump to operate the constriction device to alternately constrict and release the selected portion, such that intestinal contents is moved through the passageway of the intestines. The control device is suitably operable by the patient and preferably includes a wireless remote control.

### Electric stimulation of the intestines

An electric stimulation device may be provided for electrically stimulating muscle or neural tissue of the selected portion of the intestines to cause at least partial contraction of the selected portion. Using such a stimulation device as a complement to the constriction device enables a particularly careful treatment of the patient's intestines so that the risk of injuring the intestines over time is minimized, as will be evident from the embodiments of the invention described below.

The stimulation device may include at least one electrode adapted to stimulate muscle or neural tissue of the selected portion of the intestinal tissue with electric pulses. Preferably, the stimulation device includes a plurality of electrodes separate from or integrated with the constriction device, wherein the electrodes form a series of electrodes along the selected portion of the intestines. The electric pulses may be positive and/or negative, preferably combined positive and negative pulses. The desired stimulation effect is achieved by varying different pulse parameters, such as the pulse amplitude, the off time period between successive pulses, the pulse duration and the pulse repetition frequency. A pulse amplitude of about 5mA and a pulse duration of about 300µs are suited for neural stimulation, whereas a pulse amplitude of about 20mA and a pulse duration of about 30µs are suited for muscular stimulation. The pulse repetition frequency suitably is about 10Hz.

The control device advantageously controls the stimulation device to variably energize the electrodes along the selected portion, for example in accordance with a preset scheme, to cause partial contractions of the selected portion that over time change their positions on the selected portion, whereby parts of the intestines that currently are not stimulated can restore substantially normal blood circulation before they are stimulated again. A number or groups of the electrodes may be progressively energized in a direction upstream or downstream of the intestines. Alternatively, the electrodes may be energized one at a time in sequence or groups of the electrodes may be sequentially energized, either randomly or in accordance with a predetermined pattern.

### Pump design

Embodiments of the present invention including different conceivable designs of the pump will be described as follows.

In accordance with a simple embodiment of the invention, the constriction device of the pump includes a first constriction element for constricting and releasing the selected portion at an upstream end thereof, and a second constriction element for constricting and releasing the selected portion between the upstream and downstream ends thereof. In this embodiment, the control device controls the first and second constriction elements to alternately constrict and release the selected portion independently of one another.

For the operation of the pump, the control device is adapted to:
- control the upstream first constriction element to constrict the selected portion to close the passageway of the intestines at the upstream end of the selected portion, and
- control the second constriction element to constrict the selected portion between the upstream and downstream ends thereof to move intestinal contents contained in the selected portion downstream in the passageway of the intestines.
The control device is also adapted to:
control the first and second constriction elements to release the selected portion to allow intestinal contents in the passageway of the intestines upstream of the selected portion to enter the selected portion.

When the pump is not in operation, the stimulation device described above is used for cooperation with any of the constriction elements to close the passageway of the intestines. Thus, the first and second constriction elements are adapted to be maintained in a rest position, in which at least one of the constriction elements gently constricts the selected portion to at least substantially decrease the cross-sectional area of the passageway of the intestines, and the control device controls the stimulation device to stimulate the selected portion where the constriction element constricts the selected portion to close the passageway of the intestines. The phrase "gently constrict the selected portion" is to be understood as constricting the portion of the intestines without substantially hampering the blood circulation in intestinal tissue. The rest position allows for sufficient blood circulation in the blood vessels of the selected portion of the intestines, such that the intestinal tissues of the selected portion maintain their integrity following long exposure to the constriction element that constricts the selected portion.

The stimulation device may also be used for cooperation with any of the constriction elements when the pump is in operation. In this case, the stimulation device includes at least one electrode. Preferably, the stimulation device includes a plurality of electrodes forming a series of electrodes along a surface of at least one of the constriction elements of the constriction device, wherein the surface contacts the selected portion of the intestines. The electrodes stimulate muscle or neural tissue of the selected portion with electric pulses where one of the first constriction element and the second constriction element constricts the selected portion.

For the operation of the pump where the constriction elements and the stimulation device cooperate, the control device:
i. controls the upstream first constriction element to gently constrict the selected portion to at least substantially decrease the cross-sectional area of the passageway of the intestines at the upstream end of the selected portion,
ii. controls the stimulation device to stimulate the selected portion where the first constriction element constricts the selected portion to cause contraction of the selected portion to close the passageway of the intestines at the upstream end of the selected portion, and
iii. controls the second constriction element to constrict the selected portion between the upstream and downstream ends thereof to move intestinal contents contained in the selected portion downstream in the passageway of the intestines.
Optionally, the stimulation device may cooperate with the second constriction element by electrically stimulating the selected portion where the second constriction element constricts the selected portion to reduce the volume of the passageway of the intestines. Specifically, the control device may control the stimulation device to successively stimulate the selected portion where the second constriction element constricts the selected portion, such that the selected portion constricted by the second constriction element is progressively contracted. As a result, the intestinal contents is displaced in the passageway of the intestines in a peristaltic manner. Alternatively, the constriction device may include only a single elongated constriction element, wherein the control device controls the stimulation device to successively stimulate the selected portion where the constriction element constricts the selected portion, such that the selected portion constricted by the constriction element is progressively contracted, whereby intestinal contents is displaced in the passageway of the intestines in a peristaltic manner.

In accordance with a more sophisticated embodiment of the invention, the constriction device of the pump includes a first constriction element for constricting and releasing the selected portion at an upstream end thereof, a second constriction element for constricting and releasing the selected portion at a downstream end thereof, and a third constriction element for constricting and releasing the selected portion between the upstream and downstream ends thereof. In this embodiment, the control device controls the first, second and third constriction elements to alternately constrict and release the selected portion independently of one another.

For the operation of the pump, the control device is adapted to:
- control the upstream first constriction element to constrict the selected portion to close the passageway of the intestines at the upstream end of the selected portion,
- control the downstream second constriction element to release the selected portion, and
- control the third constriction element to constrict the selected portion between the upstream and downstream ends thereof to move intestinal contents contained in the selected portion downstream in the passageway of the intestines.

The control device is also adapted to:
- control the downstream second constriction element to constrict the selected portion to close the passageway of the intestines at the downstream end of the selected portion,
- control the upstream first constriction element to release the selected portion, and
- control the third constriction element to release the selected portion between the upstream and downstream ends thereof to allow intestinal contents in the passageway of the intestines upstream of the selected portion to enter the selected portion.

When the pump is not in operation, the stimulation device described above is used for cooperation with any of the constriction elements to close the passageway of the intestines, as described above in connection with the simple embodiment of the invention. Thus, the first, second and third constriction elements are adapted to be maintained in a rest position, in which at least one of the constriction elements gently constricts the selected portion to at least substantially decrease the cross-sectional area of the passageway of the intestines, and the control device controls the stimulation device to stimulate the selected portion where the constriction element constricts the selected portion to close the passageway of the intestines.

The stimulation device may also be used for cooperation with any of the first, second and third constriction elements when the pump is in operation. In this case, the stimulation device includes a plurality of electrodes forming a series of electrodes along a surface of at least one of the constriction elements of the constriction device, wherein the surface contacts the selected portion of the intestines. The electrodes stimulate muscle or neural tissue of the selected portion with electric pulses where one of the first constriction element and the second constriction element constricts the selected portion, and/or where the third constriction element constricts the selected portion.

For the operation of the pump where the constriction elements and the stimulation device cooperate, the control device:
- controls the upstream first constriction element to gently constrict the selected portion to at least substantially decrease the cross-sectional area of the passageway of the intestines at the upstream end of the selected portion,
- controls the stimulation device to electrically stimulate the selected portion where the first constriction element constricts the selected portion to cause contraction of the selected portion to close the passageway of the intestines at the upstream end of the selected portion,
- controls the downstream second constriction element to release the selected portion, and
- controls the third constriction element to constrict the selected portion between the upstream and downstream ends thereof to move intestinal contents contained in the selected portion downstream in the passageway of the intestines.

Optionally, the stimulation device may cooperate with the third constriction element by electrically stimulating the selected portion where the third constriction element constricts the selected portion to reduce the volume of the passageway of the intestines. Specifically, the control device may control the stimulation device to successively stimulate the selected portion where the third constriction element constricts the selected portion, such that the selected portion constricted by the third constriction element is progressively contracted. As a result, intestinal contents is displaced in the passageway of the intestines in a peristaltic manner.

Furthermore, the control device:
- controls the downstream second constriction element to gently constrict the selected portion to at least substantially decrease the cross-sectional area of the passageway of the intestines at the downstream end of the selected portion,
- controls the stimulation device to stimulate the selected portion where the second constriction element constricts the selected portion to cause contraction of the selected portion to close the passageway of the intestines at the downstream end of the selected portion,
- controls the upstream first constriction element to release the selected portion, and
- controls the third constriction element to release the selected portion between the upstream and downstream ends thereof to allow intestinal contents in the passageway of the intestines upstream of the selected portion to enter the selected portion.

In accordance with another embodiment of the invention, which includes the stimulation device, the constriction device includes a first constriction element for constricting and releasing the selected portion at an upstream end thereof, and a second constriction element for constricting and releasing the selected portion at a downstream end thereof, wherein said control device controls said first and second constriction elements to alternately constrict and release the selected portion independently of each other. In this embodiment, the stimulation device is adapted to electrically stimulate the selected portion between the upstream and downstream ends thereof to cause contraction of the selected portion to reduce the volume of the passageway of the intestines.

For the operation of the pump, the control device:
- controls the upstream first constriction element to constrict the selected portion to close the passageway of the intestines at the upstream end of the selected portion,
- controls the downstream second constriction element to release the selected portion, and
- controls the stimulation device to successively stimulate the selected portion between the upstream and downstream ends thereof to cause progressive contraction of the selected portion, so that intestinal contents is displaced in the passageway of the intestines in a peristaltic manner.

Optionally, the control device may control the upstream first constriction element to gently constrict the selected portion to at least substantially decrease the cross-sectional area of the passageway of the intestines at the upstream end of the selected portion, and control the stimulation device to stimulate the selected portion where the first constriction element constricts the selected portion to cause contraction of the selected portion to close the passageway of the intestines at the upstream end of the selected portion.

Furthermore, the control device:
- controls the downstream second constriction element to constrict the selected portion to close the passageway of the intestines at the downstream end of the selected portion,
- controls the upstream first constriction element to release the selected portion, and
- controls the stimulation device to cease stimulating the selected portion between the upstream and downstream ends thereof to allow intestinal contents in the passageway of the intestines upstream of the selected portion to enter the selected portion.

Optionally, the control device may control the downstream second constriction element to gently constrict the selected portion to at least substantially decrease the cross-sectional area of the passageway of the intestines at the downstream end of the selected portion, and control the stimulation device to stimulate the selected portion where the second constriction element constricts the selected portion to cause contraction of the selected portion to close the passageway of the intestines at the downstream end of the selected portion.

In accordance with another embodiment of the invention, the constriction device is adapted to constrict any portions of a series of selected portions of the intestines to close the passageway of the intestines, and the control device controls the constriction device to successively constrict the selected portions of the series of selected portions to move intestinal contents downstream in the passageway of the intestines in a peristaltic manner. Specifically, the constriction device includes a plurality of constriction elements, each of which is moveable along the intestines to successively constrict the selected portions of the series of selected portions, wherein the control device controls the constriction device to cyclically move the constriction elements one after the other along the selected portions of the series of selected portions. (Alternatively, the constriction device may include only one single constriction element.) Preferably, the constriction device includes a rotor carrying the constriction elements, and the control device controls the rotor to rotate, such that each constriction element cyclically constricts the selected portions of the series of selected portions. Each constriction element suitably includes a roller for rolling on the intestines to constrict the selected portions thereof.

Optionally, the stimulation device described above may be used for cooperation with the constriction device to successively constrict and contract the selected portions of the series of selected portions. Thus, the constriction device constricts any portions of the series of selected portions to at least substantially decrease the cross-sectional area of the passageway of the intestines and the stimulation device electrically stimulates the selected portion constricted by the constriction device to close the passageway of the intestines. The control device controls the constriction device to successively constrict the selected portions of the series of selected portions to move intestinal contents in the passageway of the intestines in a peristaltic manner, while controlling the stimulation device to successively stimulate the selected portions to cause successive contractions thereof in harmony with the successive constrictions of the selected portions performed by the constriction device. Specifically, the stimulation device includes one or more electrodes positioned on the constriction elements of the constriction device and adapted to stimulate intestinal tissue with electric pulses. A plurality of such electrodes may be distributed along a surface in relation to each constriction element, wherein the surface contacts the intestines as the constriction element constricts any one of the selected portions. The control device controls the constriction device to cyclically move the constriction elements one after the other along the selected portions of the series of selected portions, while controlling the stimulation device to energize the electrodes.

In accordance with another embodiment of the invention, the constriction device includes at least one elongated constriction element extending along the intestines, preferably two elongated elements extending along the intestines at opposite sides thereof. The control device controls the constriction device, such that the elongated constriction elements co-operate with each other to progressively constrict the selected portion to move intestinal contents in the passageway of the intestines. The elongated constriction elements comprise contact surfaces dimensioned for contacting a length of the selected portion of the intestines at opposite sides thereof.

The contact surfaces are suitably convex, wherein the control device controls the constriction device, such that the convex contact surfaces of the constriction elements rolls on and progressively constricts the selected portion of the intestines. Each constriction element is adapted to change between a constriction state, in which the convex surface is capable of rolling along and constricting the selected portion of the intestines, and a release state, in which the convex surface is released from the selected portion of the intestines.

Optionally, the stimulation device described above may be used for cooperation with the elongated constriction elements. Thus, the control device may control the stimulation device to stimulate the selected portion as the elongated constriction element progressively constricts the selected portion. The electrodes of the stimulation device are suitably longitudinally distributed on a surface of the elongated constriction element that contacts the selected portion of the intestines, wherein the control device controls the stimulation device to energize the electrodes successively along the elongated constriction element to cause progressive contraction of the selected portion of the intestines.

In accordance with another embodiment of the invention, said constriction device is adapted to radially expand at least a section of the selected portion of the intestines to form an expanded chamber of the passageway of the intestines along the selected portion, and to axially constrict the expanded section of the selected portion to at least substantially reduce the volume of the chamber, such that intestinal contents is displaced through the passageway of the intestines. In operation, the control device controls the constriction device to axially constrict and release the expanded section of the selected portion, so that intestinal contents is displaced through the passageway of the intestines. The constriction device is adjustable between a rest position, in which it does not expand the section of the selected portion, and an expansion position, in which it expands the section of the selected portion. Suitably, the constriction device is provided with a material that allows growth of fibrotic tissue for externally joining the expansion device with the wall of the selected portion of the intestines, whereby the expansion device pulls the wall of the selected portion radially outwardly, when it is in its expansion position, to form the expanded section of the selected portion.

Optionally, the stimulation device described above may be used for electrically stimulating the expanded section of the selected portion, when the constriction device is in its expansion position, to cause axial contraction of the expanded section of the selected portion. The electrodes of the stimulation device suitably form at least one series of electrodes extending around the expanded section of the selected portion of the intestines.

### Separate closure

In an embodiment of the invention, there is provided at least one implantable releasable closure adapted to engage the selected portion of the intestines to close the passageway of the intestines, or at least partially constrict the selected portion, when the pump is not in operation, and to release the selected portion to open the passageway of the intestines when the pump is in operation. Preferably, the closure at least partially constricts the selected portion to at least substantially decrease the cross-sectional area of the passageway of the intestines, when the pump is not in operation, and the stimulation device described above is provided to electrically stimulate muscle or neural tissue of the intestines to cause contraction of the intestines where the closure constricts the intestines to completely close the passageway of the intestines. The electrodes of the stimulation device, which are separate from or integrated with the closure, are suitably distributed along a surface of the closure that contacts the intestines. The control device controls the closure and the stimulation device to co-operate, to allow for sufficient blood circulation in the blood vessels of the constricted intestines, such that intestinal tissues maintain their integrity following long exposure to the closure, when the pump is not in operation. Where the pump includes an elongated constriction element provided with electrodes of the stimulation device, as described above, the control device may variably energize the electrodes, in order to always allow for sufficient blood circulation in the blood vessels of the constricted intestine, where the pump operates.

### Closing the passageway of the patient's intestines by the pump

Instead of providing the separate closure described above, the constriction device of the pump can be maintained in a rest position, in which it keeps the selected portion at least partially constricted, when the pump is not in operation. When the constriction device is in this rest position, it constricts the selected portion no more than to allow for sufficient blood circulation in the blood vessels of the constricted intestines, such that intestinal tissues maintain their integrity following long exposure to the constriction device. Furthermore, the constriction device constricts the intestines to at least substantially decrease the cross-sectional area of the passageway of the intestines, when the constriction device is in the rest position, and the stimulation device described above is provided to electrically stimulate the intestines where the constriction device constricts the intestines, to cause contraction of the intestines to completely close the passageway of the intestines. Preferably, the control device controls the stimulation device, to variably energize the electrodes of the stimulation device along the selected portion, to cause partial contractions of the selected portion that over time change their positions on the selected portion, whereby parts of the intestines that currently are not stimulated can restore substantially normal blood circulation before they are stimulated again.

### Artificial intestinal piece

In an embodiment of the invention, an artificial intestinal piece is surgically joined to the patient's intestines to form part of the passageway of the intestines and to form at least part of the selected portion of the intestines to be constricted by the constriction device. A significant advantage of this embodiment is that a constriction device of the various pump designs described above can be used for operating only on the artificial intestinal piece, not on the sensitive intestines. When the pump is not in operation, the constriction device can constrict the artificial intestinal piece to completely close the passageway of the intestines.

The artificial intestinal piece may be integrated with the patient's intestines between two ends thereof, for example, where a piece of the rectum has been removed due to cancer. Alternatively, the artificial intestinal piece may be joined directly or indirectly to the patient's anus.

Where the artificial intestinal piece is implanted in an ileostomy, a jejunostomy, a colostomy or a rectostomy patient, the constriction device constricts the artificial intestinal piece in order to discharge intestinal contents through a stoma located downstream of the artificial intestinal piece. Alternatively, the artificial intestinal piece may end at such a stoma.

### Manually operable pump

A subcutaneously implantable actuator operatively connected to the constriction device of the pump may be provided, wherein the actuator is manually actuatable for operating the constriction device.

In an embodiment of the invention, the constriction device is hydraulically operable, and the actuator is hydraulically connected to the hydraulically operable constriction device. The actuator preferably includes a manually compressible resilient reservoir for hydraulic fluid used for operating the constriction device.

Suitably, there is provided a reverse servo hydraulically interconnecting the reservoir and the hydraulically operable constriction device. The term "reverse servo" is to be understood as a mechanism that transfers a strong force acting on a moving element having a short stroke into a weak force acting on another moving element having a long stroke; *i.e.,* the reverse function of a normal servo mechanism. Thus, minor changes in the amount of fluid in a smaller reservoir, which in this case can be hydraulically connected to the resilient reservoir of the actuator, could be transferred by the reverse servo into major changes in the amount of fluid in a larger reservoir, which in this case can be hydraulically connected to the constriction device. The reverse servo is particularly suited for manual operation thereof.

The reservoir is hydraulically connected to the hydraulically operable constriction device, such that when the reservoir is manually compressed the constriction device constricts the selected portion between the upstream and downstream ends thereof to move intestinal contents contained in the selected portion downstream in the passageway of the intestines. When the resilient reservoir is manually released and restores its uncompressed shape, the constriction device releases the selected portion between the upstream and downstream ends thereof to allow intestinal contents in the passageway of the intestines upstream of the selected portion to enter the selected portion.

The hydraulically operable constriction device includes a constriction element and a hydraulic bellows device that operates the constriction element to constrict the selected portion between the upstream and downstream ends thereof, when the bellows device is expanded. The compressible resilient reservoir is hydraulically connected to the bellows device, such that the bellows device is expanded when the reservoir is manually compressed and retracted, when the reservoir is manually released and restores its uncompressed shape. Thus, the bellows device operates the constriction element to release the selected portion between the upstream and downstream ends, when the bellows device is retracted.

In a specific embodiment of the invention, the hydraulically operable constriction device includes a first hydraulically operable sub-device for constricting and releasing the selected portion at an upstream end thereof, a second hydraulically operable sub-device for constricting and releasing the selected portion at a downstream end thereof, and a third hydraulically operable sub-device for constricting and releasing the selected portion between the upstream and downstream ends thereof. The reservoir is hydraulically connected to the first, second and third sub-devices, such that when the reservoir is manually compressed, the upstream first sub-device constricts the selected portion to close the passageway of the intestines at the upstream end of the selected portion, the downstream second sub-device releases the selected portion, and the third sub-device constricts the selected portion between the upstream and downstream ends thereof to move intestinal contents contained in the selected portion downstream in the passageway of the intestines. When the resilient reservoir is manually released and restores its uncompressed shape, the downstream second sub-device constricts the selected portion to close the passageway of the intestines at the downstream end of the selected portion, the upstream second sub-device releases the selected portion, and the third sub-device releases the selected portion between the upstream and downstream ends thereof to allow intestinal contents in the passageway of the intestines upstream of the selected portion to enter the selected portion.

The first sub-device of the constriction device includes a first constriction element and a first hydraulic bellows device that operates the first constriction element to constrict the selected portion at the upstream end when the first bellows device is expanded, the second sub-device of the constriction device comprises a second constriction element and a second hydraulic bellows device that operates the second constriction element to release the selected portion at the downstream end when the second bellows device is expanded, and the third sub-device of the constriction device comprises a third constriction element and a third hydraulic bellows device that operates the third constriction element to constrict the selected portion between the upstream and downstream ends when the third bellows device is expanded. The compressible resilient reservoir is hydraulically connected to the first, second and third bellows devices, such that the bellows devices are expanded when the reservoir is manually compressed and retracted when the reservoir is manually released and restores its uncompressed shape. Thus, the first bellows device operates the first constriction element to release the selected portion at the upstream end when the first bellows device is retracted, the second bellows device operates the second constriction element to constrict the selected portion at the downstream end when the second bellows device is retracted, and the third bellows device operates the third constriction element to release the selected portion between the upstream and downstream ends when the third bellows device is retracted.
Where applicable, the pumps of the embodiments disclosed in this specification may be manually operable by using hydraulic means as described above.

### Powered pump

According to the invention, the pump is powered. The control device may include a manually operable switch for starting and stopping the powered pump, wherein the switch is adapted for subcutaneous implantation in the patient. Alternatively, the control device may include a wireless remote control, suitably operated by the patient holding it, for controlling the pump, *i.e*., to start and stop.

A wireless energy transmitter may be provided for transmitting wireless energy from outside the patient's body into the patient's body for powering the pump. The energy transmitter may transmit wireless energy for directly powering the pump, as the wireless energy is being transmitted. Among many things, the wireless energy may comprise electromagnetic energy, such as an electric, an electromagnetic or a magnetic field, or a combination thereof, or electromagnetic waves for direct power of the pump. For example, where the pump includes an electric pump, wireless energy in the form of a magnetic or an electromagnetic field may be used for direct power of the electric pump.

Thus, the electric pump runs directly during transmission of the wireless energy. This may be achieved in two different ways: a) using an energy-transforming device implanted in the patient to transform the wireless energy into energy of a different form, preferably electric energy, and powering the pump with the transformed energy, or b) using the wirelessly transmitted energy to directly power the pump. Preferably wireless energy in the form of an electromagnetic or magnetic field is used to directly influence specific components of the pump to create kinetic energy. Such components may include coils integrated in the pump.

Alternatively, an accumulator, such as a capacitor or a rechargeable battery, may be provided for storing the electric energy produced by the energy-transforming device, wherein the control device controls the accumulator to release energy for powering the pump. An implantable charge meter for measuring the charge of the accumulator may be provided and the control device may produce an indication in response to the charge meter.

### Sensor

In an embodiment of the invention, an implantable sensor is provided for directly or indirectly sensing a physical parameter of the patient or a functional parameter of the apparatus. Regarding the physical parameter, the control device may produce an indication, such as, a sound signal or displayed information in response to the sensor sensing a value of the physical parameter exceeding a threshold value, when the pump is not in operation. The physical parameter may be the volume of the intestinal contents in the selected portion of the intestines, the distension of the intestinal wall, or the pressure in the selected portion of the intestines.

Regarding the functional parameter of the apparatus, the control device may produce an indication, such as an alarm, a sound signal or displayed information in response to the sensor sensing a value of the functional parameter exceeding a threshold value, when the pump is in operation.

### Communication

In any of the above embodiments of the invention, there may be provided an external data communicator intended to be outside the patient's body, and an internal data communicator implantable in the patient for communicating with the external communicator. The internal data communicator feeds data related to the patient back to the external data communicator, and/or the external data communicator feeds data to the internal data communicator.

### Methods

In accordance with a second aspect of the present disclosure, there is provided a method for treating a patient having a disorder related to the passageway of the intestines. The method comprises the steps of:
a) constricting a selected portion of the patient's intestines to at least substantially reduce the volume of the passageway of the intestines along the selected portion, so that intestinal contents is displaced through the passageway of the intestines in the downstream direction thereof,
b) releasing the selected portion to increase the volume of the passageway of the intestines along the selected portion, so that intestinal contents in the passageway of the intestines upstream of the selected portion enters the selected portion, and
c) repeating steps (a) and (b) an optional number of times.

Where the selected portion of the intestines ends at the patient's anus, the method further comprises performing steps (a) and (b) to discharge intestinal contents through the anus. Alternatively, the method further comprises surgically modifying the patient's intestines to end at a stoma, and performing steps (a) and (b) to discharge intestinal contents through the stoma.

The method steps (a) and (b) can be performed in accordance with several alternatives, as will be described in the following.

Alternative 1. Step (a) is performed by initially constricting the selected portion partially to close the passageway of the intestines at an upstream end of the selected portion and then constricting the entire selected portion, and step (b) is performed by initially fully releasing the selected portion except at a downstream end thereof and then releasing the selected portion at the downstream end.

Alternative 2. Step (a) is performed by:
- partially constricting the selected portion to at least substantially decrease the cross-sectional area of the passageway of the intestines at an upstream end of the selected portion,
- electrically stimulating muscle or neural tissue of the constricted selected portion at the upstream end to cause contraction of the constricted selected portion to close the passageway of the intestines at the upstream end of the selected portion, and
- then constricting the entire selected portion.

Step (b) is performed by initially fully releasing the selected portion except at a downstream end thereof and then releasing the selected portion at the downstream end.

Alternative 3. Step (a) is performed by:
- partially constricting the selected portion to at least substantially decrease the cross-sectional area of the passageway of the intestines at an upstream end of the selected portion,
- electrically stimulating muscle or neural tissue of the constricted selected portion at the upstream end to cause contraction of the constricted selected portion to close the passageway of the intestines at the upstream end of the selected portion,
- at least partially constricting the entire selected portion, and
- then successively stimulating the selected portion from the upstream end to a downstream end thereof to cause progressive contraction of the selected portion, so that intestinal contents is displaced in the passageway of the intestines in a peristaltic manner.

Step (b) is performed by ceasing stimulating the selected portion and fully releasing the selected portion except at a downstream end thereof, and then releasing the selected portion at the downstream end.

Alternative 4. Step (a) is performed by:
- partially constricting the selected portion to at least substantially decrease the cross-sectional area of the passageway of the intestines along the entire selected portion, and
- successively stimulating the constricted portion from an upstream end to a downstream end thereof to cause progressive contraction of the selected portion, so that intestinal contents is displaced in the passageway of the intestines in a peristaltic manner.

Alternative 5. Step (a) is performed by successively constricting portions of a series of selected portions of the intestines so that intestinal contents is displaced downstream in the passageway of the intestines in a peristaltic manner.

Alternative 6. Step (a) is performed by:
- successively constricting portions of a series of selected portions of the intestines so that each constricted portion at least substantially decreases the cross-sectional area of the passageway of the intestines, and
- electrically stimulating each constricted portion to cause contraction of the constricted intestinal portion to close the passageway of the intestines, whereby intestinal contents is displaced downstream in the passageway of the intestines in a peristaltic manner.

Alternative 7. Step (a) is performed by progressively constricting the selected portion so that intestinal contents is displaced downstream in the passageway of the intestines in a peristaltic manner.

Alternative 8. Step (a) is performed by:
- electrically stimulating the selected portion to cause contraction thereof, and
- progressively constricting the contracted selected portion so that intestinal contents is displaced downstream in the passageway of the intestines in a peristaltic manner.

The present disclosure also provides another method for treating a patient having a disorder related to the passageway of the intestines. This method comprises the steps of:
- providing a pump including a constriction device engaging a selected portion of the intestines to constrict and release the selected portion,
- controlling the pump to operate the constriction device to alternately constrict the selected portion to at least substantially reduce the volume of the passageway of the intestines along the selected portion and release the selected portion to increase the volume of the passageway of the intestines along the selected portion, so that intestinal contents is displaced through the passageway of the intestines.

This method further comprises providing at least one releasable closure engaging the patient's intestines, and using the closure to at least substantially close the passageway of the intestines, when the pump is not in operation, and to release the intestines, when the pump is in operation. The closure may be used to constrict the intestines to close the passageway of the intestines.

Alternatively, the closure may be used to constrict the intestines to at least substantially decrease the cross-sectional area of the passageway of the intestines, wherein the method further comprises electrically stimulating muscle or neural tissue of the intestines to cause contraction of the intestines where the closure constricts the intestines to close the passageway of the intestines. In this case, the method suitably comprises coordinating the operation of the closure and the electric stimulation of the intestines, to allow for sufficient blood circulation in the blood vessels of the constricted intestines, so that intestinal tissues maintain their integrity following long exposure to the closure, when the pump is not in operation.

The present disclosure also provides a method (I) for using an apparatus as described above to treat a patient having a disorder related to the passageway of the intestines. This method (I) comprises the steps of:
- providing an apparatus as described above,
- inserting a needle like tube into the abdomen of the patients body,
- supplying gas through the tube to fill the abdomen with gas, thereby expanding the abdominal cavity,
- placing at least two laparoscopical trocars in the patient's body,
- inserting through one of the trocars a camera into the abdomen,
- selecting a portion of the patient's intestines,
- inserting a dissecting tool through one of the trocars and dissecting an area of the selected portion,
- placing the pump of the apparatus in the dissected area in operative engagement with the selected portion of the intestines, and
- using the pump to pump intestinal contents through the passageway of the intestines. The method (I) may further comprise cutting an opening through the patient's skin and abdominal wall and passing the patient's intestine through the opening creating a stoma, and selecting the portion of the patient's intestines in the vicinity of the stoma, wherein the pump is used for discharging the intestinal contents through the stoma.

The present disclosure provides another method (II) for using an apparatus as described above to treat a patient having a disorder related to the passageway of the intestines. This method (II) comprises the steps of:
- providing an apparatus as described above,
- cutting an opening through the patient's skin and abdominal wall,
- selecting a portion of the patient's intestines,
- inserting a dissecting tool through the opening and dissecting an area of the selected portion,
- placing the pump of the apparatus in the dissected area in operative engagement with the selected portion of the intestines, and
- using the pump to pump intestinal contents through the passageway of the intestines.

The present disclosure provides yet another method (III) for using an apparatus as described above to treat a patient having a disorder related to the passageway of the intestines. This method (III) comprises the steps of:
- cutting an opening through the patient's skin and abdominal wall and passing a portion of the patient's intestines through the opening creating a stoma,
- cutting the intestines to separate a short piece of the intestines forming the stoma from the remaining part of the intestines while keeping blood vessels of the mesentery connected to the short piece of the intestines, to ensure blood supply to the short piece of the intestines,
- dissecting an area at the short piece of the intestines,
- placing an artificial intestinal piece in the dissected area, and surgically joining it to the short piece of the intestines and to the remaining part of the intestines to form a continuous passageway of the intestines through the remaining part of the intestines, the artificial intestinal piece and the short piece of the intestines, the artificial intestinal piece forming a selected portion of the patient's intestines,
- providing an apparatus as described above,
- placing the pump of the apparatus in operative engagement with the artificial intestinal piece, and
- using the pump to pump intestinal contents through the passageway of the intestines and out of the stoma.

The above methods (I), (II) and (III) may further comprise subcutaneously implanting a manually operable switch for starting and stopping the pump.

In accordance with the above methods (I), (II) and (III), the constriction device of the pump is put in operative engagement with the selected portion of the intestines, and the pump is controlled to operate the constriction device to alternately constrict the selected portion to at least substantially reduce the volume of the passageway of the intestines along the selected portion, and release the selected portion to increase the volume of the passageway of the intestines along the selected portion, so that intestinal contents is displaced through the passageway of the intestines.

The methods (I), (II) and (III) further comprise implanting at least one releasable closure in operative engagement with the selected portion of the intestines, and using the releasable closure to close the passageway of the intestines when the pump is not in operation and to release the intestines when the pump is in operation.

The methods (I), (II) and (III) further comprise implanting an electrically powered operation device, such as an electric motor, for operating the releasable closure. Alternatively, the operation device includes a hydraulic operation device.

The methods (I), (II) and (III) further comprise transmitting wireless energy for powering the operation device, and when desired to pump intestinal contents through the passageway of the intestines, powering the operation device with the transmitted energy to operate the closure to release the selected portion of the intestines.

The methods (I), (II) and (III) further comprise implanting a source of energy in the patient, providing an external source of energy, controlling the external source of energy to release wireless energy, transforming the wireless energy into storable energy, non-invasively charging the implanted source of energy with the transformed energy, and controlling the implanted source of energy from outside the patient's body to release energy for use in connection with the operation of the pump and/or releasable closure. The patient's intestines may be surgically modified to end in a stoma, and when desired to discharge intestinal contents out from the patient's body, the implanted source of energy is controlled to supply energy for operating the releasable closure to temporarily release the selected portion of the intestines and the pump is used to pump intestinal contents in the passageway of the intestines out from the body through the stoma. The wireless energy may be transformed into a storable energy, such as electric energy, different than the wireless energy, wherein the storable energy is used for operating the pump and/or releasable closure.

Alternatively, the methods (I), (II) and (III) further comprise providing an external source of energy outside the patient's body, controlling the external source of energy from outside the patient's body to release wireless energy, and using the released wireless energy for operating the pump and/or releasable closure. For example, the external source of energy may be controlled to release wireless energy directly operating the pump and/or releasable closure. The external source of energy may be controlled to release non-magnetic wireless energy, wherein the released non-magnetic wireless energy is used for operating the pump and/or releasable closure. Alternatively, the external source of energy may be controlled to release electromagnetic wireless energy, wherein the released electromagnetic wireless energy is used for operating the pump and/or releasable closure.

The wireless energy may be transformed into electrical energy inside the patient's body by an implanted energy-transforming device, wherein the electrical energy is used in connection with the operation of the pump and/or releasable closure. The electrical energy may be directly used in connection with the operation of the pump and/or releasable closure. For example, the pump and/or releasable closure may be directly operated with the electrical energy, as the energy-transforming device transforms the wireless energy into the electrical energy.

The methods (I), (II) and (III) further comprise controlling the releasable closure to:
- at least partially restrict the passageway of the intestines in the selected portion to prevent intestinal contents from passing therethrough, or
- release the intestines to allow intestinal contents to be pumped therethrough by using the pump.

Step (a) may be performed by controlling the releasable closure to partially restrict the passageway of the intestines in the selected portion and electrically stimulating the selected portion of the intestines to cause contraction thereof to further restrict the passageway of the intestines to prevent intestinal contents from passing therethrough.

The present disclosure provides yet another method for using an apparatus, as described above, to treat a patient having a disorder related to the passageway of the intestines. This method comprises:
- providing a wireless remote control adapted to control the pump from outside the patient's body, and
- operating the wireless remote control by the patient to start the pump, when the patient wants to defecate, and stop the pump when the patient has finished defecating.

### BRIEF DESCRIPTION OF THE DRAWINGS

FIGURES 1A and 1B schematically illustrate different stages of operation of a general apparatus according to the present invention, wherein a pump of the apparatus is applied on a patient's intestines.
FIGURES 2A and 2B schematically illustrate different stages of operation of the general apparatus of FIGURES 1A and 1B also including an electric stimulation device.
FIGURES 3A, 3B and 3C schematically illustrate different stages of operation of an embodiment of the present invention, wherein a peristaltic pump is applied on a patient's intestines.
FIGURES 4A, 4B and 4C schematically illustrate the embodiment of FIGURES 3A-3C also including an electric stimulation device.
FIGURES 5A, 5B and 5C are longitudinal cross-sections of an embodiment of the invention showing different stages of operation, wherein a pump includes a constriction device that radially constricts a patient's intestines.
FIGURES 6A, 6B and 6C are longitudinal cross-sections of a modification of the embodiment of FIGURES 5A - 5C, including an electric stimulation device.
FIGURE 7 shows the same embodiment as that of FIGURE 6C illustrating a modified operation of the stimulation device.
FIGURE 8 shows the embodiment of FIGURE 6A when the pump is not in operation.
FIGURES 9A, 9B, 9C and 9D illustrate a modified operation of the stimulation device of the embodiment according to FIGURE 6A when the pump is not in operation.
FIGURES 10A, 10B and 10C are longitudinal cross-sections of another embodiment of the invention including an electric stimulation device and showing different stages of operation.
FIGURES 11A and 11B are views of another embodiment of the invention showing different stages of operation, wherein a rotary peristaltic pump is applied on a patient's small intestines.
FIGURES 12A and 12B are views of a modification of the embodiment of FIGURES 11A and 11B, also including an electric stimulation device.
FIGURES 13A through 13D are longitudinal cross-sections of another embodiment of the invention showing different stages of operation, wherein another type of peristaltic pump is applied on a patient's small intestines.
FIGURES 14A through 14D are longitudinal cross-sections of a modification of the embodiment of FIGURES 13A-13D, including an electric stimulation device.
FIGURE 15A and 15B show different stages of operation of another embodiment of the invention including a separate intestinal closure.
FIGURE 16A shows another embodiment of the invention including an artificial intestinal piece implanted in a colostomy patient, wherein a pump operates on the artificial intestinal piece.
FIGURE 16B shows the artificial intestinal piece of FIGURE 16A joined to the patient's anus.
FIGURE 17 shows an enlarged detail of the embodiment of FIGURE 16.
FIGURE 18 shows a modification of the embodiment of FIGURE 17.
FIGURES 19A, 19B, 19C and 19D schematically illustrate different stages of operation of another embodiment of the invention, wherein a pump includes a constriction device that axially constricts a patient's intestines.
FIGURE 20 illustrates the embodiment of FIGURE 19D also including an electric stimulation device.
FIGURE 21 is a side view of another embodiment of the invention, wherein a pump includes a hydraulically operable constriction device applied on a patient's intestines in a first stage of operation.
FIGURES 22 and 23 are views taken along section lines XXII-XXII and XXIII-XXIII, respectively, in FIGURE 21.
FIGURE 24 is a side view of the embodiment of FIGURE 21 with the constriction device in a second stage of operation.
FIGURES 25 and 26 are views taken along section lines XXV-XXV and XXVI-XXVI, respectively, in FIGURE 24.
FIGURES 27A and 27B show different stages of operation of a hydraulic reverse servo suited for connection with the hydraulic constriction device of FIGURES 21 - 26.
FIGURES 28A and 28B show different stages of operation of a mechanically operated constriction device suited for use in some of the embodiments of the present invention.
FIGURE 28C is a modification of the embodiment of FIGURES 28A and 28B.
FIGURE 29 illustrates the pump of the embodiment of FIGURES 6A and 6B applied on the small intestines of a colostomy patient.
FIGURE 30 illustrates the pump of the embodiment of FIGURES 6A and 6B applied on a colostomy patient's small intestines ending at the patient's anus.

### DETAILED DESCRIPTION OF PREFERRED EMBODIMENTS

Referring to the drawing figures, like reference numerals designate identical or corresponding elements throughout the several figures.

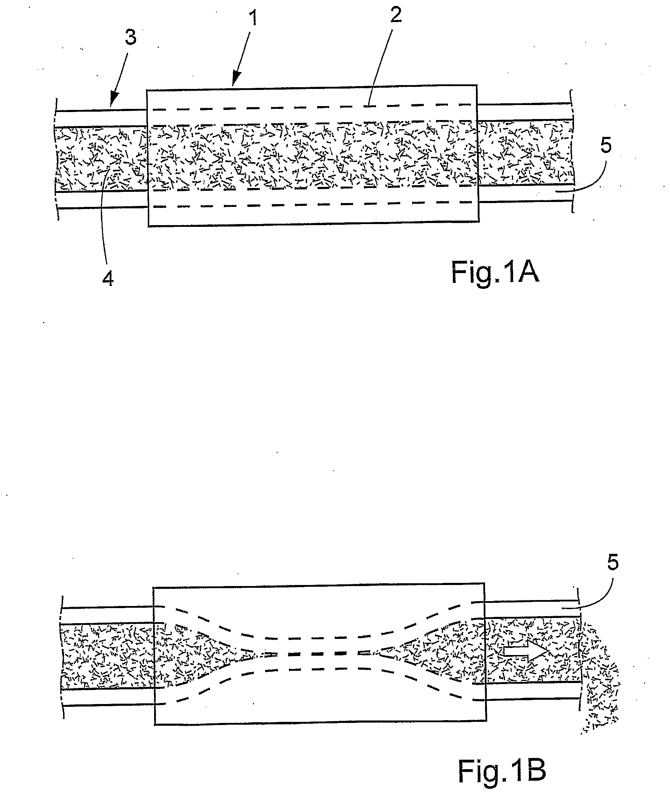

FIGURE 1A schematically shows a general embodiment of the apparatus according to the present invention for treating a patient suffering from a disorder related to the passageway of the patient's intestines, such as constipation or anal incontinence. The apparatus includes a pump 1 externally applied on a selected portion 2 of the patient's intestines 3, such as a portion of the large intestine. The pump 1 includes a constriction device for constricting the selected portion 2 to displace intestinal contents therein. In operation, the apparatus cyclically changes between a first stage of operation, in which the constriction device does not constrict the selected portion 2 to allow intestinal contents to fill the selected portion 2, as illustrated in FIGURE 1A, and a second stage of operation, in which the constriction device constricts the selected portion 2 to at least substantially reduce the volume of the passageway of the intestines 4 along the selected portion 2, so that the selected portion 2 of the tubular intestine 3 is at least partially flattened. As a result, intestinal contents is displaced out of the selected portion 2 downstream in the intestine 3 and discharges through an open end 5 of the intestine 3, as illustrated in FIGURE 1B. When the apparatus changes from the second stage of operation (FIGURE 1B) to the first stage of operation (FIGURE 1A), the constriction device of the pump 1 releases the selected portion 2 to allow intestinal contents to enter the selected portion 2, whereby the volume of the passageway of the intestines 4 along the selected portion 2 is increased as intestinal contents fills the selected portion 2.

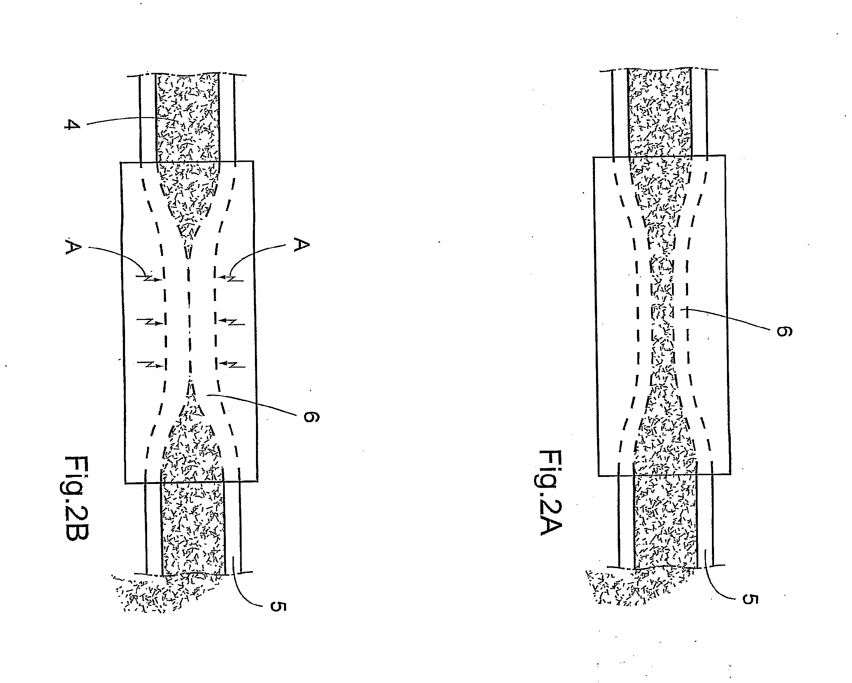

The apparatus of FIGURE 1A may be provided with an electric stimulation device for electrically stimulating muscle or neural tissue of the selected portion 2 to cause contraction of the intestinal wall 6, so that the intestinal wall 6 thickens. When operating the apparatus provided with such a stimulation device, the apparatus cyclically performs a sequence of three stages of operation, namely a first stage of operation, in which the constriction device of the pump 1 does not constrict the selected portion 2 to allow intestinal contents to fill the selected portion 2, as illustrated in FIGURE 1A, a second stage of operation, in which the constriction device constricts the selected portion 2 so that the latter is partially flattened, as illustrated in FIGURE 2A, whereby some intestinal contents is displaced downstream in the intestine 3 and discharges through the open end 5, and a third stage of operation, in which the electric stimulation device stimulates the constricted selected portion 2 with electric pulses (indicated by arrows A in FIGURE 2B) to thicken the intestinal wall 6 to completely close the passageway of the intestines 4, whereby more intestinal contents is displaced downstream in the intestine 3 and discharges through the open end 5, see FIGURE 2B. The use of the electric stimulation device for accomplishing complete closing of the passageway of the intestines 4 enables the constriction device of the pump 1 to partially constrict the selected portion 2 without substantially hampering the blood circulation in the intestinal tissue.

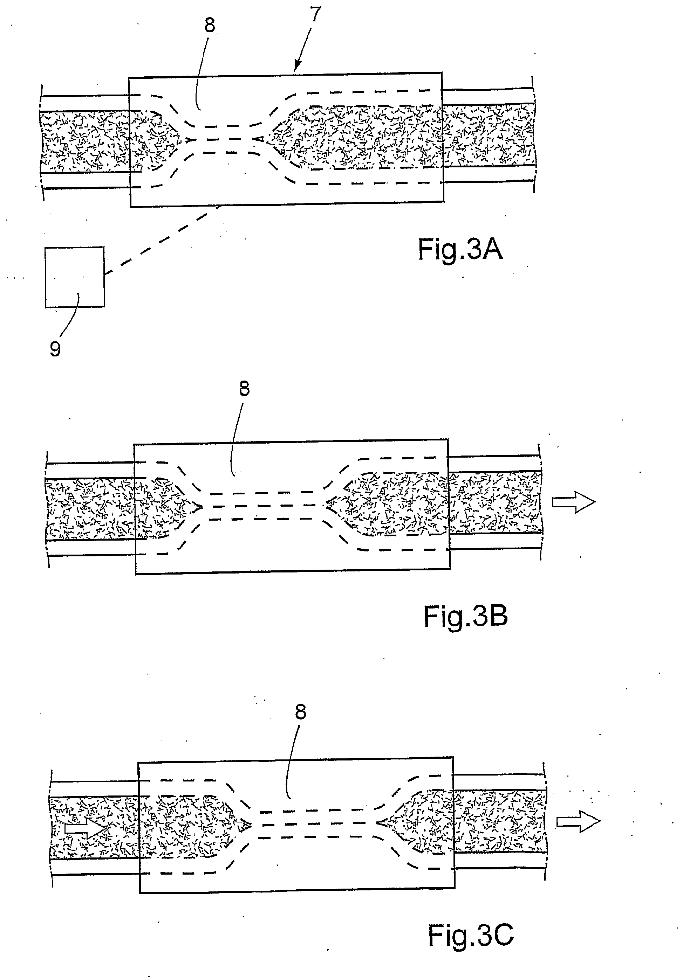

FIGURES 3A, 3B and 3C schematically illustrate an embodiment of the invention, which is similar to the general embodiment according to FIGURES 1A and 1B, except that the apparatus includes a peristaltic type of pump 7. The peristaltic pump 7 includes a constriction device that provides a limited constriction 8 of the selected portion 2 extending along a part of the selected portion 2, see FIGURE 3A. A control device 9 is provided for controlling the constriction device of the peristaltic pump 7 to displace the constriction 8 in the downstream direction (from left to right in FIGURES 3A-3C) to move intestinal contents forwards in the passageway of the intestines 4, as illustrated in FIGURES 3A through 3C. When the constriction 8 has been displaced to the right end of the selected portion 2 and intestinal contents has entered and refilled the selected portion 2 upstream of the constriction 8, the control device 9 controls the constriction device of the peristaltic pump 7 to release the selected portion 2 at its right end and to apply the constriction 8 at the left end of the selected portion 2, as illustrated in FIGURE 3a, whereby the above described operation can be repeated.

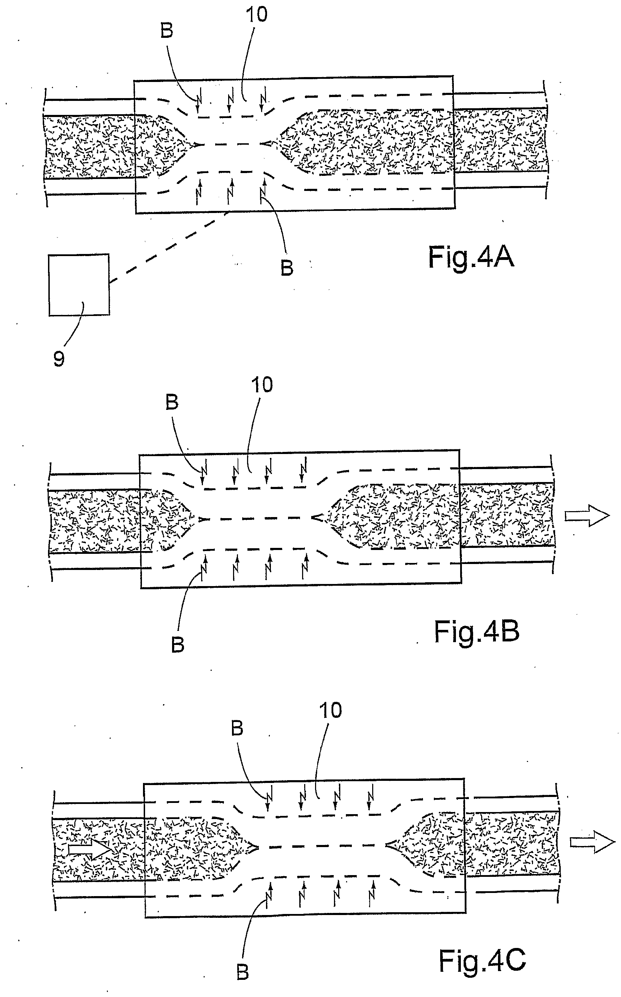

FIGURES 4A, 4B and 4C schematically illustrate an embodiment of the invention, which is similar to the embodiment according to FIGURES 3A, 3B and 3C, except that the apparatus of the embodiment of FIGURES 3A-3C also includes an electric stimulation device having the same purpose as the electric stimulation device described above in connection with the embodiment according to FIGURES 2A and 2B. In the embodiment of FIGURES 4A-4C, a limited constriction 10 of the selected portion 2 is provided by the combination of two measures. Thus, in accordance with a first measure, the constriction device of the peristaltic pump 7 constricts a part of the selected portion 2 so that the latter partially flattens but does not close the passageway of the intestines 4. In accordance with a following second measure, the electric stimulation device stimulates the constricted part of the selected portion 2 with electric pulses (indicated by arrows B in FIGURES 3A-3C) to thicken the intestinal wall to completely close the passageway of the intestines, whereby the constriction 10 is created. The control device 9 controls the constriction device and stimulation device to displace the constriction 10 in the same manner as described above for the corresponding constriction 8 according to the embodiment of FIGURES 3A-3C.

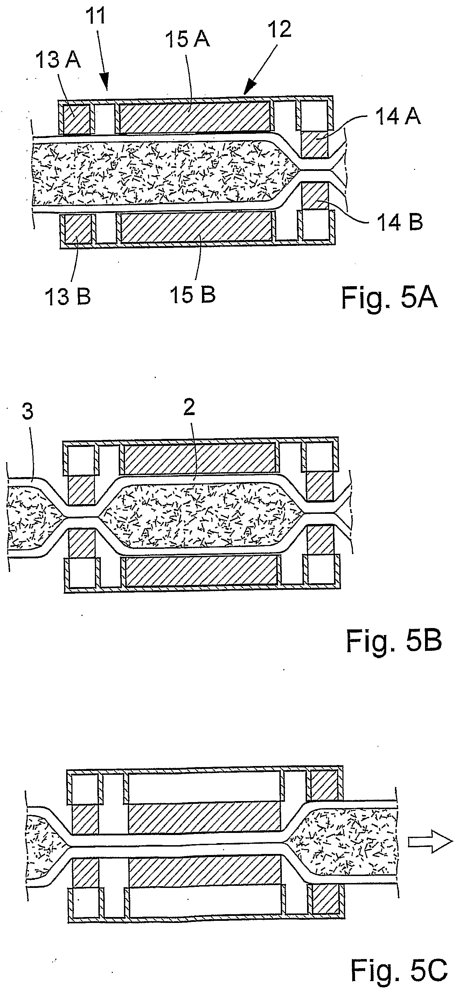

FIGURES 5A, 5B and 5C show an embodiment of the invention, in which the apparatus includes a pump 11 having a constriction device 12 designed to constrict, *i.e.,* flatten a selected portion 2 of the patient's intestines 3. Thus, the constriction device 12 includes an upstream first pair of short constriction elements 13A and 13B, a downstream second pair of short constriction elements 14A and 14B, and a third pair of elongate constriction elements 15A and 15B positioned between the first and second short element pairs. The two short constriction elements 13A,13B of the first pair are radially movable towards and away from each other between retracted positions (FIGURE 5A) and constricting positions (FIGURES 5B and 5C), the two short constriction elements 14A, 14B of the second pair are radially movable towards and away from each other between retracted positions (FIGURE 5C) and constricting positions (FIGURES 5A and 5B), and the two elongate constriction elements 15A, 15B of the third pair are radially movable towards and away from each other between retracted positions (FIGURES 5A and 5B) and constricting positions (FIGURE 5C). The pump 11 is applied to the selected portion 2 so that the two constriction elements of each pair of constriction elements are positioned at opposite sides of the selected portion 2, the short constriction elements 13A, 13B being positioned at an upstream end of the selected portion 2, and the short constriction elements 14A, 14B being positioned at a downstream end of the selected portion 2.

The control device 9 controls the pair of short constriction elements 13A, 13B, the pair of elongate constriction elements 15A, 15B and the pair of short elements 14A, 14B to constrict and release the selected portion 2 independently of one another. FIGURES 5A - 5C illustrate how the control device 9 controls the operation of the pump 11 to cyclically displace intestinal contents in the downstream direction of the passageway of the intestines 4. Thus, in FIGURE 5A the short constriction elements 13A, 13B and the elongate constriction elements 15A, 15B are in their retracted positions, whereas the short constriction elements 14A, 14B are in their constricting positions. FIGURE 5B illustrates how the short constriction elements 13A, 13B have also been moved radially inwardly to their constricting positions, whereby a volume of intestinal contents is trapped in the passageway of the intestines 4 between the upstream and downstream ends of the selected portion 2. FIGURE 5C illustrates how the short constriction elements 14A, 14B initially have been moved radially outwardly to their retracted positions, and then the elongate constriction elements 15A, 15B have been moved radially inwardly to their constricting positions. As a result, the intestinal contents in the passageway of the intestines 4 between the upstream and downstream ends of the selected portion 2 has been moved downstream in the passageway of the intestines 4, as indicated by an arrow. Then, the control device 9 controls the constriction device 12 to assume the state shown in FIGURE 5A to allow intestinal contents to enter and fill the passageway of the intestines between the upstream and downstream ends of the selected portion 2, whereby the pumping cycle is completed.

Although FIGURES 5A - 5C disclose pairs of constriction elements, it should be noted that it is conceivable to design the constriction device 12 with only a single short constriction element 13A, a single elongate constriction element 15A and a single short constriction element 14A. In such a case, the selected portion 2 of the intestine 3 is supported by stationary elements of the constriction device 12 positioned at the side of the selected portion 2 which is opposite to the constriction elements 13A, 14A and 15A.

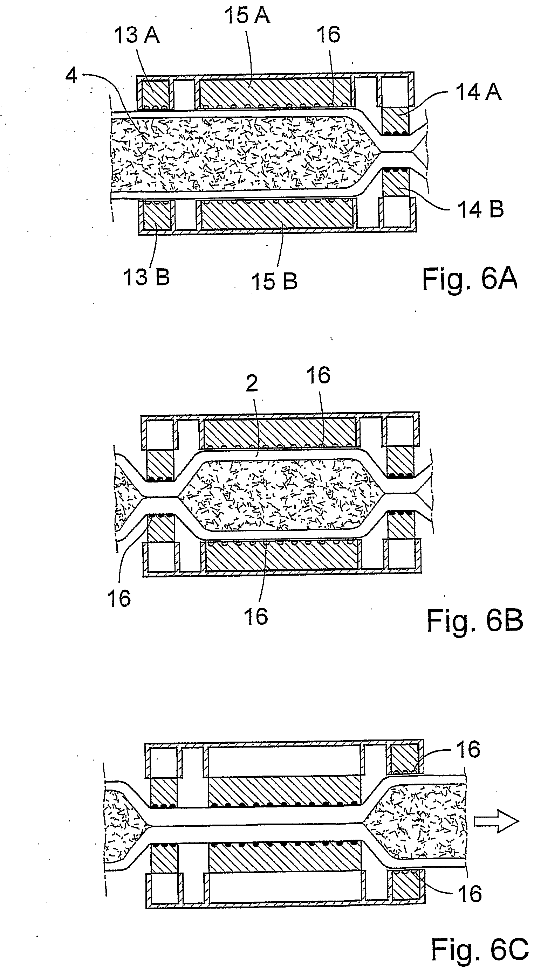

FIGURES 6A, 6B and 6C show an embodiment of the invention, which is similar to the embodiment according to FIGURES 5A, 5B and 5C, except that the apparatus according to FIGURES 6A-6C also includes an electric stimulation device having the same purpose as the electric stimulation device described above in connection with the embodiment according to FIGURES 2A and 2B. The stimulation device according to FIGURES 6A-6C includes rows of electrodes 16 positioned on the constriction elements 13A, 13B, 14A, 14B, 15A and 15B and adapted to stimulate muscle or neural tissue of the selected portion of the intestinal tissue with electric pulses. In FIGURES 6A-6C, inactivated electrodes 16 are indicated by unfilled rings, whereas activated electrodes 16 are indicated by black round spots. In this embodiment, the constriction device and stimulation device cooperate to avoid hampering the blood circulation in the intestinal tissue. Thus, the two constriction elements of each pair of constriction elements are adapted to constrict the selected portion no more than to almost close the passageway of the intestines. The final complete closure of the passageway of the intestines is achieved by the electrodes 16 stimulating the constricted selected portion 2 with electric pulses, so that the intestinal wall thickens and completely closes the passageway of the intestines.

In operation of the apparatus according to FIGURES 6A-6C, the control device 9 controls the constriction elements 13A-15B to move in the same sequence as described above in connection with the embodiment according to FIGURES 5A-5C. The control device 9 also controls the electrodes 16 to electrically stimulate the intestinal tissue where any one of the constriction elements 13A-15A constricts the selected portion 2.

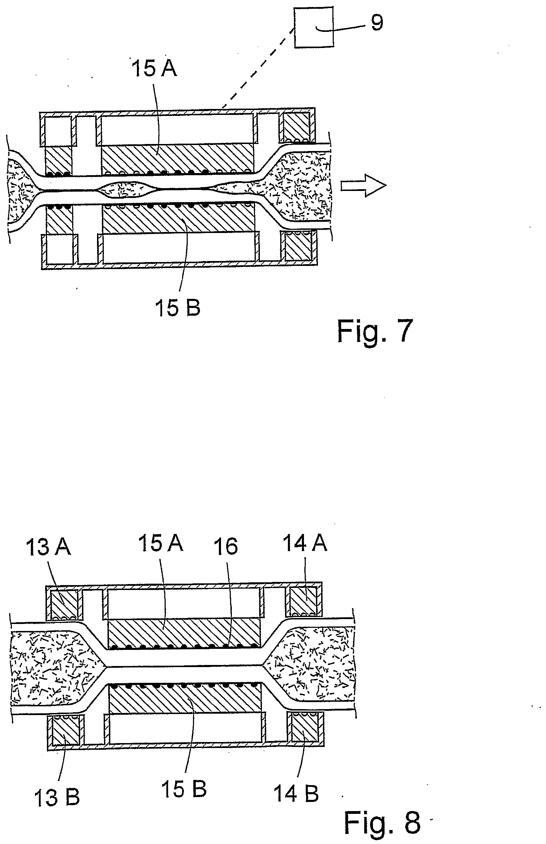

FIGURE 7 shows the same embodiment as that of FIGURE 6C illustrating a modified operation of the electrodes 16 on the elongate constriction elements 15A and 15B. Thus, the control device 9 controls the electrodes 16 to successively stimulate the selected portion 2 where the elongate constriction elements 15A, 15B constrict the selected portion 2, so that the constricted selected portion is progressively contracted in the downstream direction. As a result, intestinal contents is displaced downstream in the passageway of the intestines in a peristaltic manner. Alternatively, the electrodes 16 may successively stimulate the selected portion 2 to progressively contract the portion 2 in the upstream direction.

FIGURE 8 shows the embodiment of FIGURE 6A when the pump 11 is not in operation and the constriction elements 13A-15B are maintained in a rest position. Thus, the short upstream and downstream constriction elements 13A, 13B, 14A and 14B are kept in their retracted positions, whereas the elongate constriction elements 15A,15B gently constrict the selected portion to at least substantially decrease the cross-sectional area of the passageway of the intestines. The control device 9 controls all of the electrodes 16 on the constriction elements 15A, 15B to electrically stimulate the constricted selected portion 2 to thicken the intestinal wall so that the passageway of the intestines is kept completely closed. Alternatively, only a part of the constricted portion may be stimulated at a time.

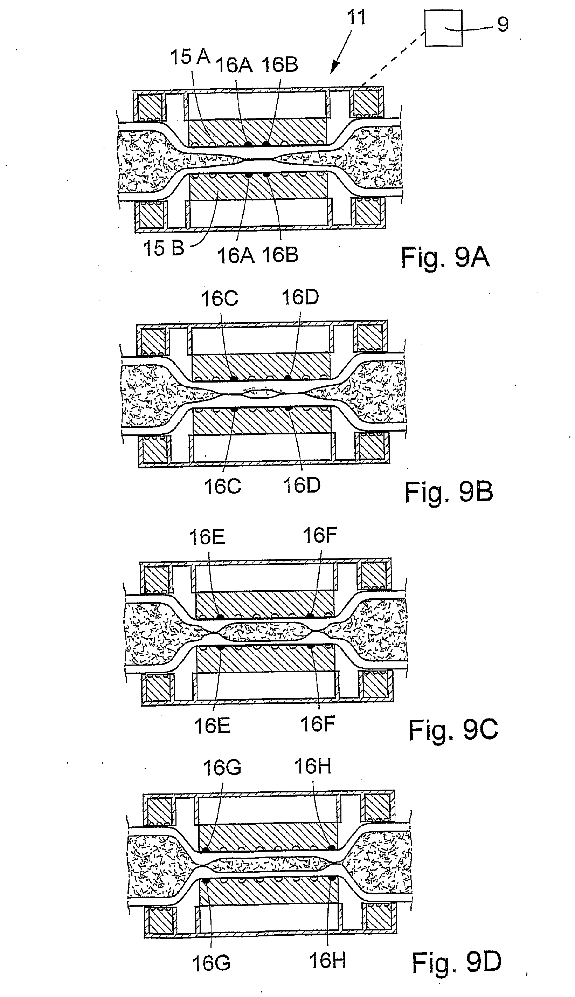

FIGURES 9A, 9B, 9C and 9D illustrate a modified operation of the electrodes 16 of the embodiment according to FIGURE 6A when the pump 11 is not in operation. Referring to FIGURE 9A, there are eight electrodes positioned in a row along constriction element 15A and eight electrodes positioned in a row along constriction element 15B. The control device 9 activates the electrodes in the two rows of electrodes in accordance with a preset scheme to cause partial contractions of the selected portion 2 that over time change their positions on the selected portion, whereby parts of the intestines that currently are not stimulated can restore substantially normal blood circulation before they are stimulated again. Starting from the electrodes positioned in the middle of the elongate constriction elements 15A, 15B, the electrodes are progressively activated in the direction upstream and the direction downstream of the intestines.

Thus, the control device 9 activates two pairs of adjacent electrodes 16A and 16B positioned centrally in the two rows of electrodes to thicken the intestinal wall along a short distance of the selected portion 2 to close the passageway of the intestines in the middle of the selected portion 2. Referring to FIGURE 9B, after the laps of a predetermined time period, in the order of seconds, a next pair of electrodes 16C to the left of the electrode pair 16A and a next pair of electrodes 16D to the right of the electrode pair 16B are activated, whereas the electrodes 16A and 16B are inactivated. As a result, the intestinal wall is thickened at two different positions spaced from the middle of the selected portion 2. Referring to FIGURE 9C, after the laps of another predetermined time period, a next pair of electrodes 16E to the left of the electrode pair 16C and a next pair of electrodes 16F to the right of the electrode pair 16D are activated, whereas the electrodes 16C and 16D are inactivated. As a result, the intestinal wall is thickened at two positions spaced farther from the middle of the selected portion 2. Referring to FIGURE 9D, after the laps of yet another predetermined time period, a next pair of electrodes 16G to the left of the electrode pair 16E and a next pair of electrodes 16H to the right of the electrode pair 16F are activated, whereas the electrodes 16E and 16F are inactivated. Then, the above described stimulation operation according to FIGURES 9A-9D is cyclically repeated until the pump 11 is to be operated.

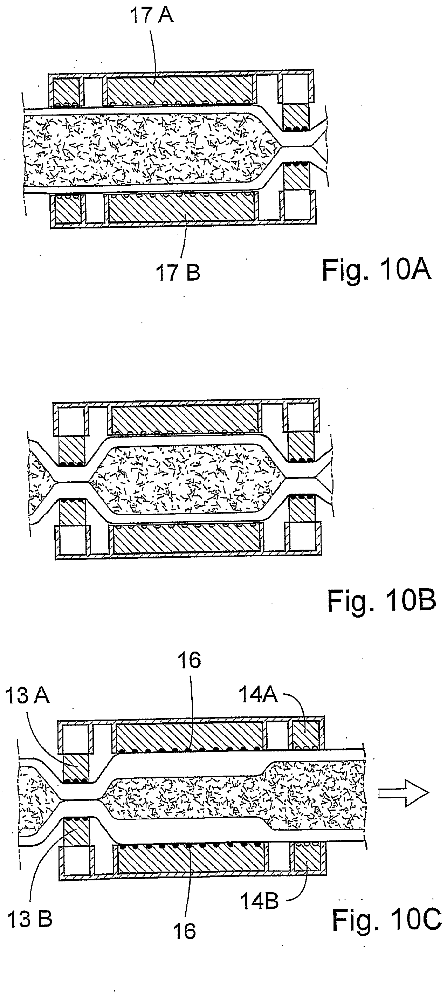

FIGURES 10A, 10B and 10C show an embodiment of the invention, which is similar to the embodiment according to FIGURES 6A, 6B and 6C, except that the movable elongate constriction elements 15A and 15B are replaced by two elongate stationary elements 17A and 17B. The stimulation device of this embodiment includes rows of electrodes 16 positioned on the stationary elements 17A, 17B and adapted to electrically stimulate the intestinal wall of the selected portion 2 to reduce the volume of the passageway of the intestines 4 between the upstream and downstream ends of the selected portion 2. Thus, FIGURE 10C illustrates how the short constriction elements 14A, 14B initially have been moved radially outwardly to their retracted positions, and then the electrodes 16 have been activated to cause contraction of the intestinal wall so that the volume of the passageway of the intestines between the upstream and downstream ends of the selected portion 2 is reduced, whereby intestinal contents is moved downstream in the passageway of the intestines 4 as indicated by an arrow. For the sake of clarity, the thickness of the intestinal wall subjected to electric stimulation by the electrodes 16 is exaggerated in FIGURE 10C.

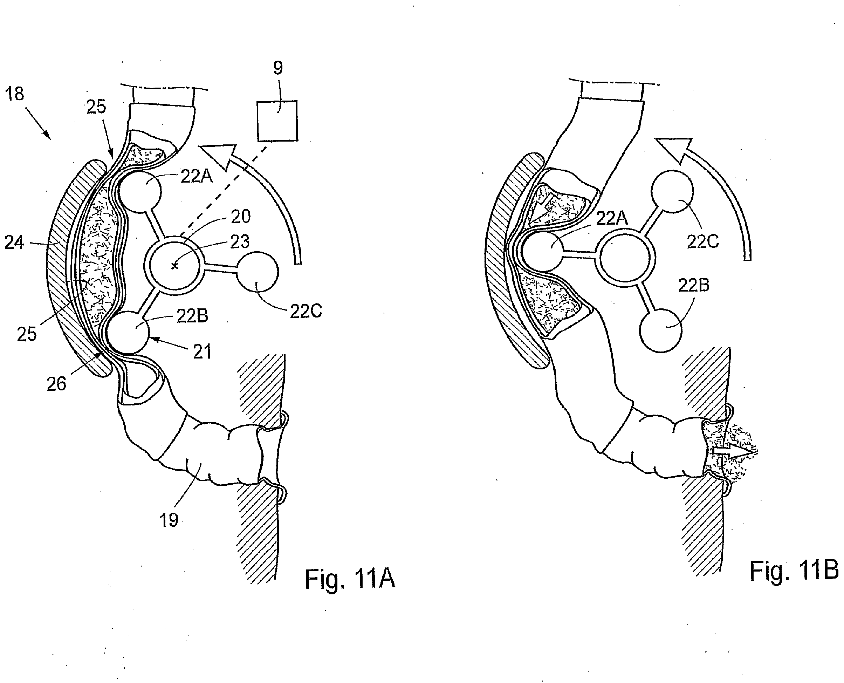

FIGURES 11A and 11B are views of another embodiment of the invention showing different stages of operation, wherein a rotary peristaltic pump 18 is applied on the small intestines 19 of a colostomy patient near the stoma. The peristaltic pump 18 includes a rotor 20 carrying a constriction device 21 in the form of three cylindrical constriction elements 22A, 22B and 22C positioned equidistantly from the axis 23 of the rotor 20. The constriction elements 22A-22C may be designed as rollers. A stationary elongate support element 24 is positioned spaced from but close to the rotor 20 and has a part cylindrical surface 25 concentric with the axis 23 of the rotor 20. The pump 18 is applied on the small intestines 19, so that the intestine 19 extends between the support element 24 and the rotor 20.

The control device 9 controls the rotor 20 to rotate so that the constriction elements 22A-22C successively constrict portions of a series of selected portions of the intestines 19 against the elongate support element 24. FIGURE 11A illustrates how the constriction element 22A constricts intestines 19 at a first portion 25 and closes the passageway of intestines 4, whereas the constriction element 22B is about to release intestine 19 at a second portion 26 downstream of the first portion 25. FIGURE 11B illustrates how the constriction element 22A has advanced about halfway along the elongate support element 24 and displaced the intestinal contents in the passageway of the intestines so that some intestinal contents discarges through the stoma. The constriction element 22B has released intestines 19, whereas the constriction element 22C is about to engage intestines 19. Thus, the control device 9 controls the rotor 20 to cyclically move the constriction elements 22A-22C one after the other along the elongate support element 24 while constricting the selected portions of intestines 19, so that intestinal contents in the passageway of intestines 4 is displaced in a peristaltic manner. The same constriction principle may also be practised by other mechanical constriction devices that do not include a rotor.

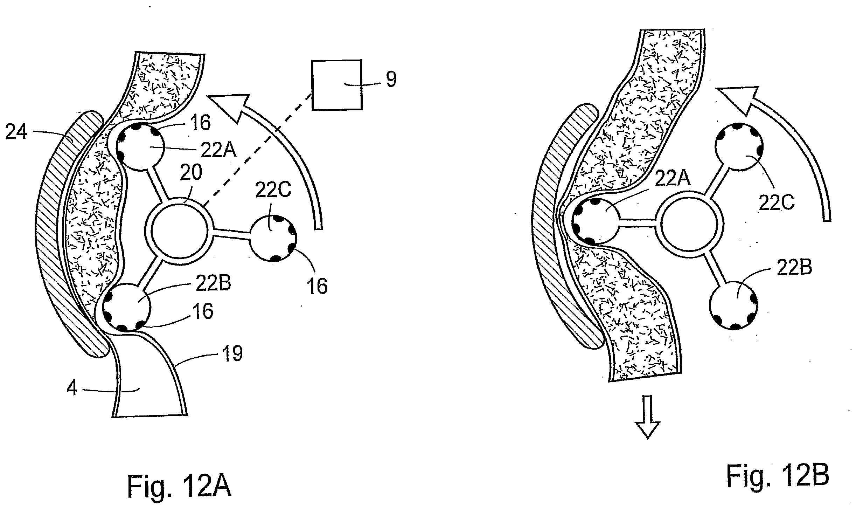

FIGURES 12A and 12B show an embodiment of the invention, which is similar to the embodiment according to FIGURES 11A and 11B, except that the apparatus of the embodiment of FIGURES 12A-12B also includes an electric stimulation device having the same purpose as the electric stimulation device described above in connection with the embodiment according to FIGURES 4A-4C. The stimulation device according to FIGURES 12A-12B includes electrodes 16 provided on the constriction elements 22A-22C. In this embodiment, the rotor 20 is spaced somewhat farther from the stationary elongate support element 23, as compared with the embodiment according to FIGURES 11A and 11B, so that the constriction elements 22A-22C do not completely close the passageway of the intestines by mechanical action, as they constrict intestine 19 during rotation of the rotor 20. Complete closure of the passageway of intestines 4 is accomplished by activation of the electrodes 16. The same combined constriction and stimulation principle may also be practised by other mechanical constriction devices that do not include a rotor.

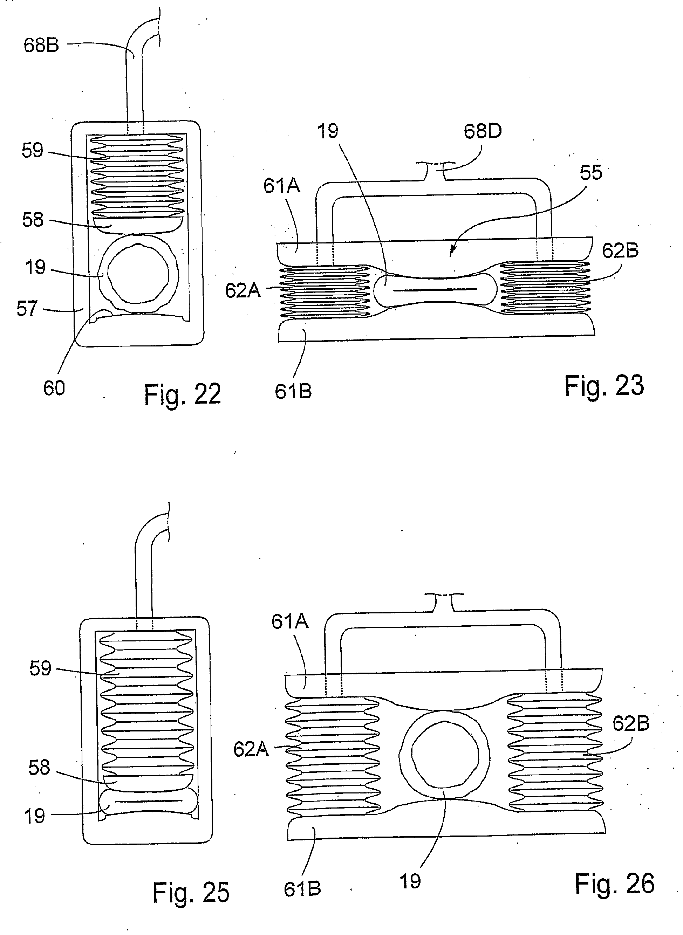

Thus, when any one of the constriction elements 22A-22C constricts a portion of intestines 19, its associated electrodes 16 electrically stimulate the constricted portion with electric pulses so that the intestinal wall of the constricted portion thickens and closes the passageway of intestines 4. FIGURE 22A illustrates how the constriction element 22A has started to constrict a portion of intestines 19 and how the passageway of intestine 4 is closed with the aid of the activated electrodes 16 on the constriction element 22A.

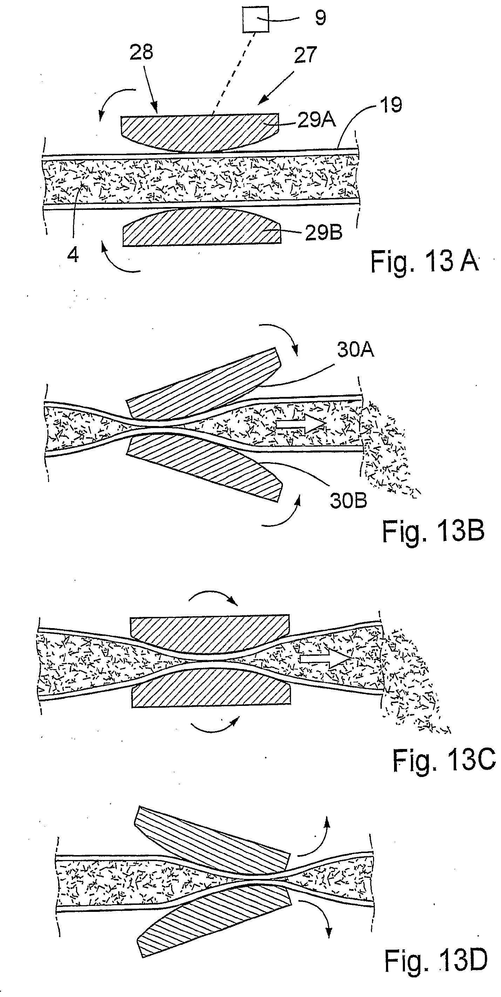

FIGURES 13A through 13D are longitudinal cross-sections of another embodiment of the invention showing different stages of operation, wherein another type of peristaltic pump 27 is applied on a selected portion of the patient's intestines 19. The pump 27 comprises a constriction device 28 including two elongate constriction elements 29A and 29B having convex surfaces 30A and 30B that abut a length of the selected portion on mutual sides thereof. The control device 9 controls the elongate constriction elements 29A, 29B to move relative to the selected portion so that the constriction elements 29A, 29B progressively constrict the selected portion, as shown in FIGURES 13A to 13D.

Thus, in an initial position of the constriction elements 29A, 29B shown in FIGURE 13A, the selected portion is not constricted by the constriction elements 29A, 29B. Starting from this initial position, the control device 9 controls the constriction elements 29A, 29B to swing the left ends of the constriction elements 29A, 29B toward the selected portion (indicated by arrows) to constrict the selected portion of intestines 19, see FIGURE 13A. FIGURE 13B shows how the passageway of intestines 4 is completely closed by the constricted selected portion. Then, as shown in FIGURE 13B, the control device 9 controls the constriction elements 29A, 29B to move so that their right ends move towards each other (indicated by arrows), while the convex surfaces 30A, 30B of the constriction elements 29A, 29B are rolling on each other with the constricted selected portion between them, see FIGURE 13C. As a result, intestinal contents in intestines 19 is forced to the right (indicated by a white arrow). When the constriction elements 29A, 29B have rolled on each other to the position shown in FIGURE 13D, the control device 9 controls the constriction elements 29A, 29B to move their right ends away from each other (indicated by arrows in FIGURE 13D) to the initial position shown in FIGURE 13A. The operation stages shown in FIGURES 13A to 13D can be cyclically repeated a number of times until the desired amount of intestinal contents has been displaced in the passageway of the intestines in a peristaltic manner. This embodiment is particularly suited for use in a stoma patient. Thus, the peristaltic pump 27 is applied on the patient's intestines close to the patient's stoma.

Alternatively, only one of the constriction elements 29A, 29B can be provided with a convex surface, whereas the other constriction element has a plane surface that abuts the selected portion. It is also possible to use a single constriction element with a convex surface that presses the selected portion of the intestine 19 against a bone of the patient.

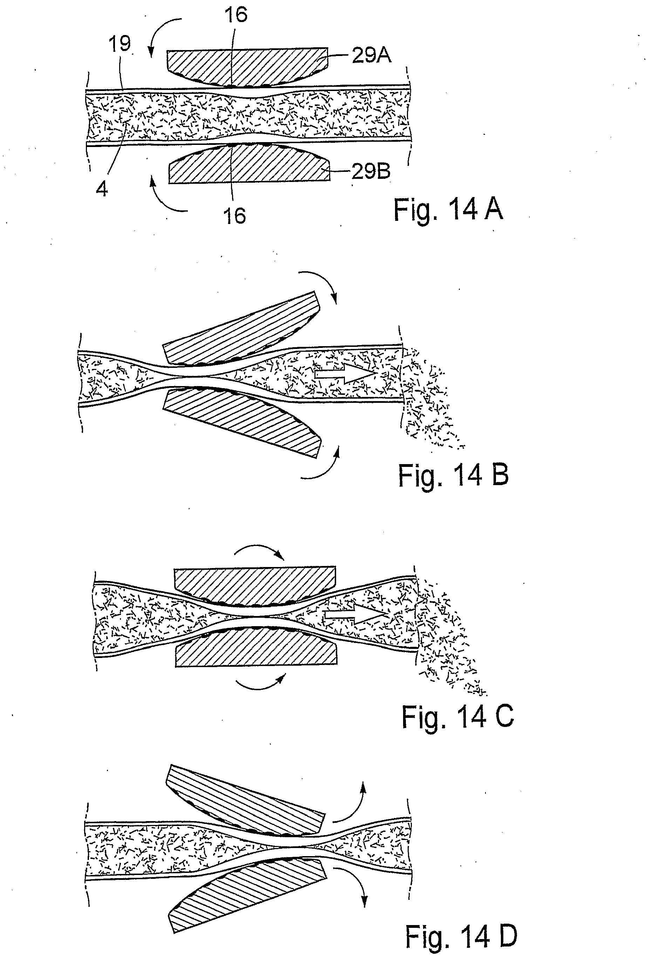

FIGURES 14A through 14D show an embodiment of the invention, which is similar to the embodiment according to FIGURES 13A - 13D, except that the apparatus of the embodiment of FIGURES 14A-14D also includes an electric stimulation device having the same purpose as the electric stimulation device described above in connection with the embodiment according to FIGURES 4A-4C. The stimulation device includes electrodes 16 positioned on the convex surfaces 30A, 30B. In the embodiment of FIGURES 14A - 14D, the constriction elements 29A and 29B are spaced somewhat farther from each other, as compared with the embodiment according to FIGURES 13A - 13D, so that the constriction elements 29A, 29B do not completely close the passageway of the intestines 4 by mechanical action, as they constrict the intestine 19 during operation. Complete closure of the passageway of the intestines 4 is accomplished by activation of the electrodes 16.

Thus, with the constriction elements 29A, 29B in the initial position shown in FIGURE 14A, the control device 9 controls the constriction elements 29A, 29B to swing the left ends thereof toward the selected portion of the intestines 19 (indicated by arrows) to constrict the selected portion, while controlling the electrodes 16 to electrically stimulate the intestine 19 to cause contraction and thickening of the intestinal wall. FIGURE 14B shows how the passageway of the intestines 4 is completely closed by the combination of the mechanical constriction of the intestine 19 by the constriction elements 29A, 29 B and the electric stimulation of the intestine 19 by the electrodes 16. FIGURES 14C and 14D shows operation stages that correspond to the operation stages of FIGURES 13C and 13D described above.

In the embodiment according to FIGURES 14A to 14D, the control device 9 may control the electrodes 16 to progressively stimulate the constricted selected portion to cause progressive contraction thereof in harmony with the movement of the elongate constriction elements 29A 29B, as the convex surfaces 30A, 30B of the constriction elements 29A, 29B are rolling on each other.

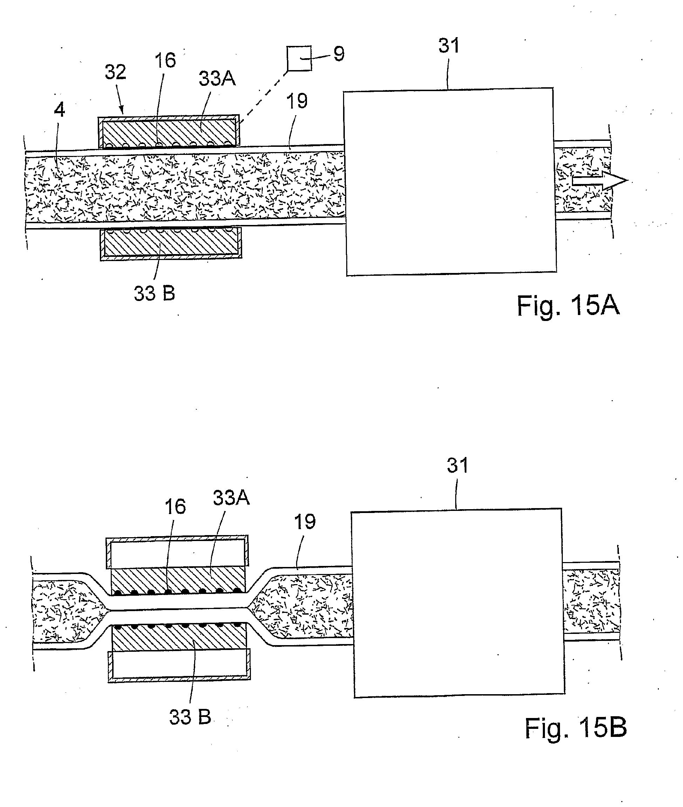

FIGURES 15A and 15B show different stages of operation of another embodiment of the invention, in which the apparatus includes a separate pump 31 and a separate releasable closure 32 applied on a selected portion of a patient's intestine 19. The pump 31 is of the type described above, which includes a constriction device that alternately constricts and releases the selected portion of the intestines 19, such that intestinal contents is displaced through the passageway of the intestines 4. The closure 32 includes two elongate constriction elements 33A and 33B, which are positioned at opposite sides of the selected portion. The constriction elements 33A, 33B are radially movable towards and away from each other between retracted positions (FIGURE 15A), in which the selected portion of the intestine 19 is released when the pump 31 is in operation, and constricting positions (FIGURE 5C), in which the constriction elements 33A, 33B constrict the selected portion to close the passageway of the intestines when the pump is not in operation.

There is a stimulation device including rows of electrodes 16 positioned on the constriction elements 33A, 33B for stimulating muscle or neural tissue of the selected portion of the intestine 19 with electric pulses to cause contraction of the intestinal wall. FIGURE 15A illustrates the electrodes 16 by unfilled rings indicating inactivated electrodes and FIGURE 15B illustrates the electrodes 16 by black round spots indicating activated electrodes. The constriction elements 33A, 33B and electrodes 16 cooperate to avoid hampering the blood circulation in the intestinal tissue, when the closure 32 closes the passageway of the intestines 4. Thus, the constriction elements 33A, 33B at least partially constrict the selected portion to at least substantially decrease the cross-sectional area of the passageway of the intestines 4, when the pump 31 is not in operation (FIGURE 15B), and the electrodes electrically stimulate muscle or neural tissue of the intestines to cause contraction of the intestines, so that the intestinal wall thickens, to completely close the passageway of the intestines 4. The electrodes may be activated in accordance with the preset scheme described above in connection with the embodiment of FIGURES 9A - 9D, or in accordance with any other determined pattern or scheme that causes variation of the stimulation of the intestine.

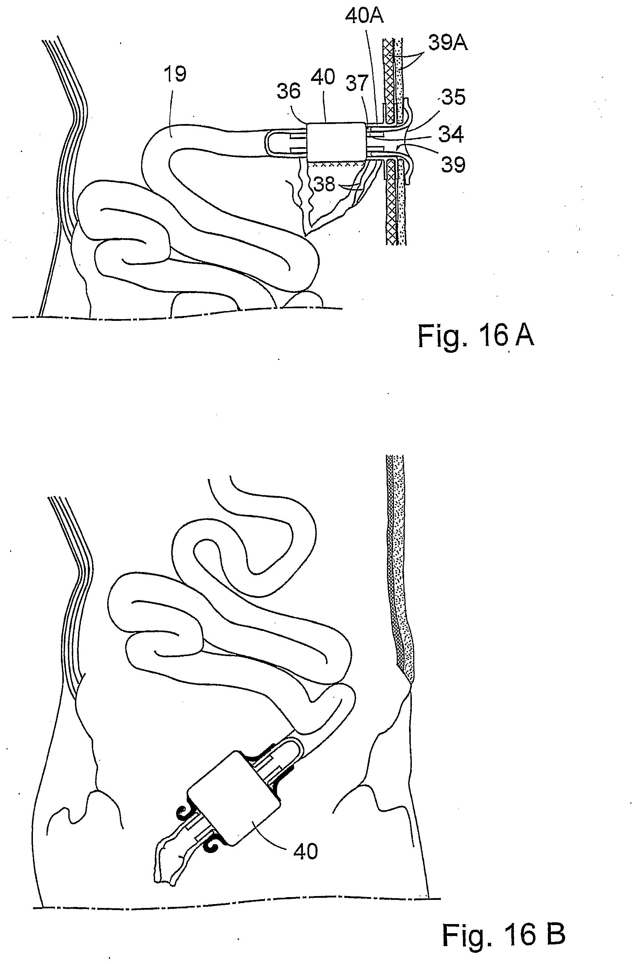

FIGURE 16A shows another embodiment of the invention including an artificial intestinal piece 34 implanted in a colostomy patient provided with a stoma 35. The small intestines 19 is surgically cut to form an upstream open end 36 thereof and a downstream open end 37 of a short separated piece 39 of the small intestines 19 that forms the stoma 35. The short piece 39 of the intestines 19 extends through a surgically created opening in the patient's abdominal wall 39A. The artificial intestinal piece 34 is surgically joined to and integrated with the patient's small intestines 19 between the upstream end 36 and downstream end 37. Blood vessels 38 of a portion of the patient's mesentery supply blood to the separate piece 39 of the intestines that forms the stoma 35. The artificial intestinal piece 34 is provided with a pump 40, which includes a constriction device that only operates on the artificial intestinal piece 34. A holder 40A attached to the pump 40 is secured to the abdominal wall 39A at the opening thereof. The constriction device of pump 40 alternately constricts and releases the artificial intestinal piece 34, which may include an elastic tubing, so that intestinal contents is discharged through the patient's stoma 35. The constriction device of pump 40 may be selected from any one of the various constriction devices described in the embodiments of the present application. In the embodiment of FIGURE 16A, the constriction device may constrict the artificial intestinal piece 34 to normally keep the passageway thereof closed, when the pump 40 is not in operation. Also, any kind of pump that is capable of constricting the small intestines may be used in the embodiment of FIGURE 16A.

The artificial intestinal piece 34 or 43 described above may alternatively be joined directly or indirectly to the patient's anus, as illustrated in FIGURE 16B.

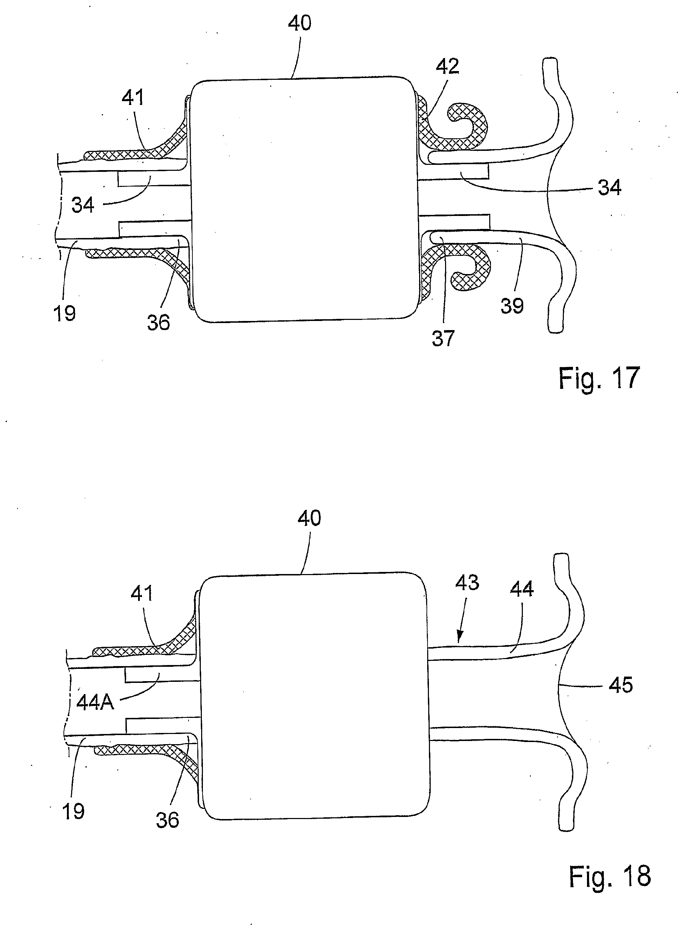

FIGURE 17 is an enlarged view of the artificial intestinal piece 34 and the pump 40 showing how the intestinal piece 34 with the pump 40 is sealed to the intestines 19 at the upstream and downstream ends 36, 37 thereof. Thus, a fabric tubular net 41 is applied on the intestines 19 at the upstream end 36 thereof and is attached to the pump 40. Another fabric tubular net 42 is applied on the separate piece 39 of the intestines 19 and is attached to the pump 40. The tubular net 42 has not been fully applied on the separate piece 39 to illustrate how the net 42 is rolled on the piece 39. Initially the fabric nets 41 and 42 are sutured to the intestines, normally with absorbable sutures. The tubular nets 41, 42 promote ingrowth of fibrotic tissue that seals the intestines 19 to the artificial intestinal piece 34. Another material such as PTFE, silicone or polyurethane may be applied externally on the tubular nets 41, 42.

FIGURE 18 shows a modification of the embodiment of FIGURE 17, which includes an artificial intestinal piece 43 having a tubular portion 44 that extends from the pump 40 at the downstream side thereof and forms a stoma 45. Another tubular portion 44A of the artificial intestinal piece 43 extends from the pump 40 at the upstream end thereof. With this modification there is only need for surgically joining and sealing the tubular portion 44A of the artificial intestinal piece 43 to the patient's small intestines 19.

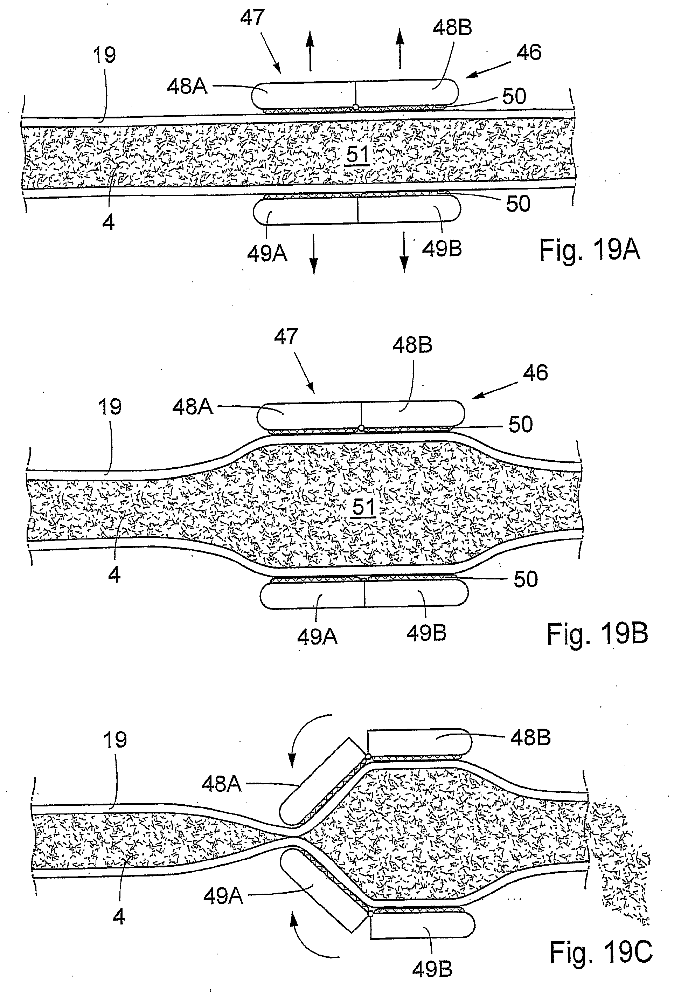

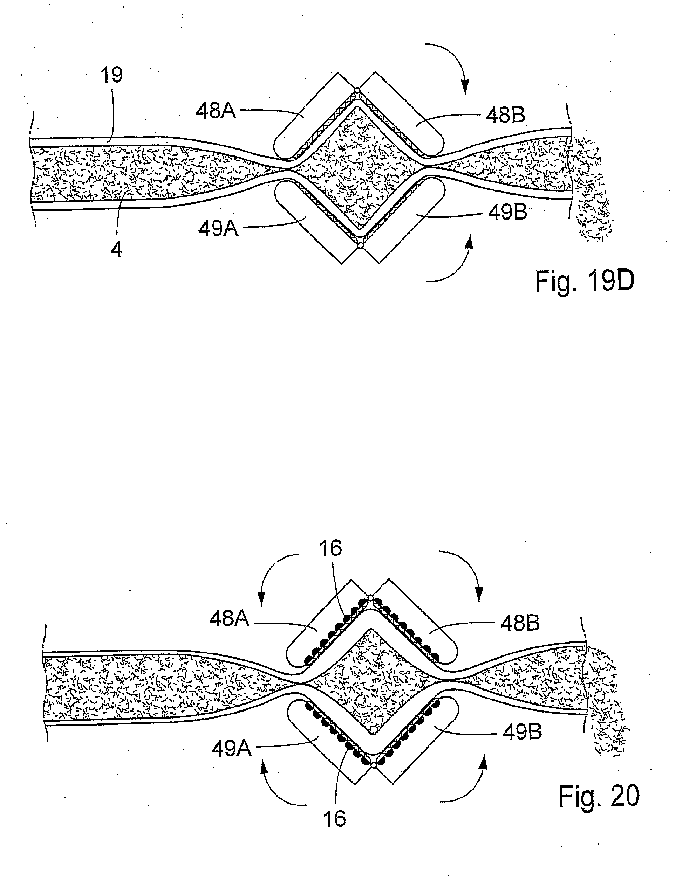

FIGURE 19A, 19B, 19C and 19D schematically illustrate different stages of operation of another embodiment of the invention, wherein a pump 46 includes a constriction device 47 that axially constricts a patient's intestines 19. Referring to FIGURE 19A, the constriction device 47 includes a first pair of constriction elements 48A and 48B hinged to each other and a second pair of constriction elements 49A and 49B hinged to each other. The two pairs of constriction elements 48A, 48b and 49A, 49B are joined to a selected portion of the intestines 19 at opposite sides thereof by means of a material 50 that allows ingrowth of fibrotic tissue. FIGURE 19B shows how the first pair of constriction elements 48A, 48B and the second pair of constriction elements 49A, 49B have been moved away from each other to radially expand the selected portion of intestines 19 to form an expanded chamber 51 of the passageway of intestines 4. FIGURE 19C shows how the constriction elements 48A and 49B are turned towards each other to radially and axially constrict the selected portion of intestines 19, whereby the volume of the chamber 51 is reduced causing intestinal contents to displace through the passageway of the intestines and out of intestines 19. FIGURE 19D shows how also the constriction elements 48B and 49B of the second pair are turned towards each other, to axially constrict the expanded selected portion to further reduce the volume of the chamber 51, whereby more intestinal contents is displaced through the passageway of intestines 4 and out of intestines 19.

FIGURE 20 illustrates the embodiment of FIGURE 19D including an electric stimulation device having the same purpose as the electric stimulation device described above in connection with the embodiment according to FIGURES 4A-4C. The stimulation device of FIGURE 20 includes electrodes 16 positioned on the constriction elements 48A, 48B, 49A and 49B. The electrodes 16 electrically stimulate the intestines 19 to cause contraction and thickening of the intestinal wall at the same time as the constriction elements 48A, 48B, 49A and 49B axially constricts the intestines 19.

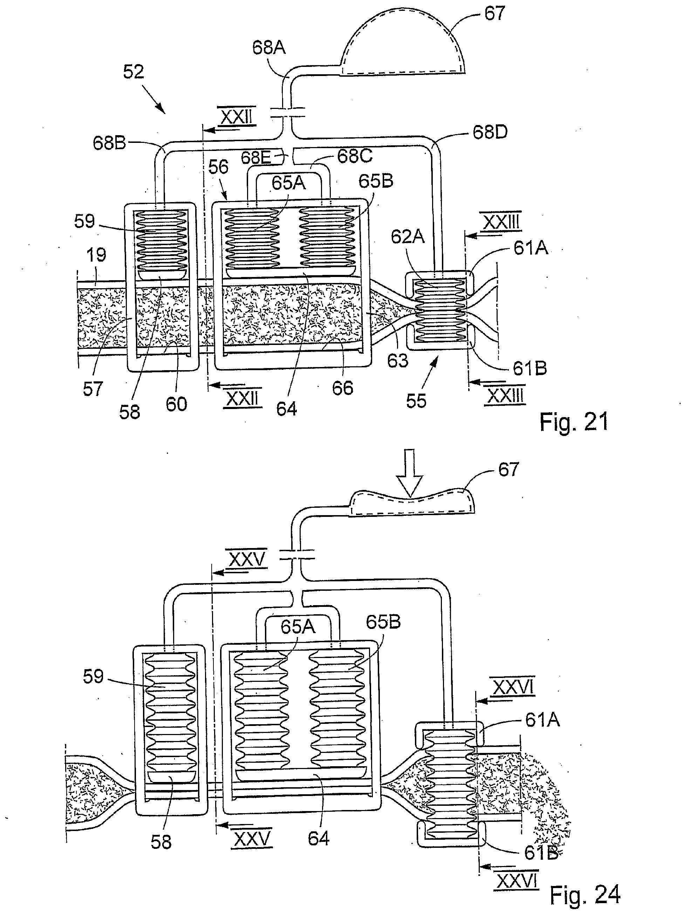

FIGURES 21 - 26 show another embodiment of the disclosure, wherein a manually operable pump 52 includes a hydraulically operated constriction device 53 applied on a selected portion of the patient's intestines 19. The constriction device 53 includes a first sub-device 54 for constricting and releasing the selected portion at an upstream end thereof, a second sub-device 55 for constricting and releasing the selected portion at a downstream end thereof, and a third sub-device 56 for constricting and releasing the selected portion between the upstream and downstream ends thereof. The first sub-device 54 includes a frame 57, a constriction element 58 movable relative to the frame 57 and a hydraulic bellows 59 connected between the frame 57 and the constriction element 58. A support surface 60 of the frame 57 supports the intestine 19, so that the constriction element 58 can constrict the intestine 19 against the support surface 60, see FIGURE 22. The second sub-device 55 includes two constriction elements 61A and 61B positioned at opposite sides of the intestine 19 and two bellows 62A and 62B also positioned at opposite sides of the intestine 19 and interconnecting the constriction elements 61A, 61B, see FIGURE 23. The third sub-device 56 is designed similar to the first sub-device 54 and includes a frame 63, an elongate constriction element 64 movable relative to the frame 63 and two hydraulic bellows 65A and 65B connected between the frame 63 and the elongate constriction element 64. A support surface 66 of the frame 63 supports the intestine 19. The bellows 59, 65A, 65B, 62A, 62B are dimensioned, such that when they are fully expanded, the volume of the bellows 59 is equal to the volume of the two bellows 62A and 62B, whereas the volume of the two bellows 65A and 65B is larger than the volume of the bellows 59 and larger than the volume of the bellows 62A and 62B.

An actuator in the form of a manually compressible elastic reservoir 67 containing a volume of hydraulic fluid is subcutaneously implantable in the patient's body and hydraulically connected to the respective bellows 59, 65A, 65B, 62A and 62B via hydraulic conduits 68A, 68B, 68C, 68D and 68E of equal size. Conduit 68C branches hydraulic fluid supplied through the single conduit 68E to the two bellows 65A and 65B. FIGURES 21 -23 show the pump 52 in an inactivated state, in which the reservoir 67 is uncompressed, whereby all of the bellows 59, 65A, 65B, 62A, 62B are retracted. As a result, the sub-device 55 constricts the intestine 19 at the downstream end of the selected portion (see FIGURE 23), whereas the sub-devices 54 and 56 release the intestine 19.

FIGURES 24 - 26 show the pump 52 in an activated state, in which the patient manually compresses the reservoir 67 to distribute hydraulic fluid from the reservoir 67 to the bellows 59, 65A, 65B, 62A, 62B. As a result, the bellows 59 expands so that the short constriction element 58 constricts the intestine 19 and closes the passageway of the intestines 4 at the upstream end of the selected portion of the intestine 19 (see FIGURE 25), the two bellows 62A, 62B expand so that the short constriction elements 61A, 61B release the intestine 19 and open the passageway of the intestines 4 at the downstream end of the selected portion (see FIGURE 26), and the two bellows 65A, 65B expand so that the elongate constriction elements 64 constrict the selected portion between the upstream and downstream ends thereof, whereby intestinal contents is displaced in the passageway of the intestines 4. Since the bellows 59 has a smaller volume when expanded than the combined volume of the two bellows 65A, 65B and hydraulic fluid is supplied to the bellows 65A, 65B via the single conduit 68E, the bellows 59 expands more quickly than the bellows 65A, 65B, when the reservoir 67 is compressed. (This difference in the rate of expansion may alternatively be achieved by designing bellows 59 with a smaller volume than the volume of each one of bellows 65A and 65B, and connecting bellows 65A to conduit 68B and bellows 65B to conduit 68D.) Consequently, the constriction element 58 closes the passageway of the intestines 4 at the upstream end of the selected portion of the intestine 19 well before the elongate constriction element 64 fully constricts the intestines, whereby a significant amount of intestinal contents in the selected portion of the intestine 19 is forced downstream in the passageway of the intestines as the constriction element 64 constricts the intestine 19. When the patient ceases to compress the reservoir 67, the reservoir resumes its uncompressed shape sucking hydraulic fluid from the bellows 59, 65A, 65B, 62A, 62B, whereby the bellows 59, 65A, 65B, 62A, 62B retract and the pump 52 returns to the inactivated state shown in FIGURE 21.

The hydraulic operation means of the constriction device according to FIGURES 21 - 23 described above may also be implemented in the constriction devices of the embodiments according to FIGURES 1- 10C and in the closure of the embodiment according to FIGURES 15A, 15B.

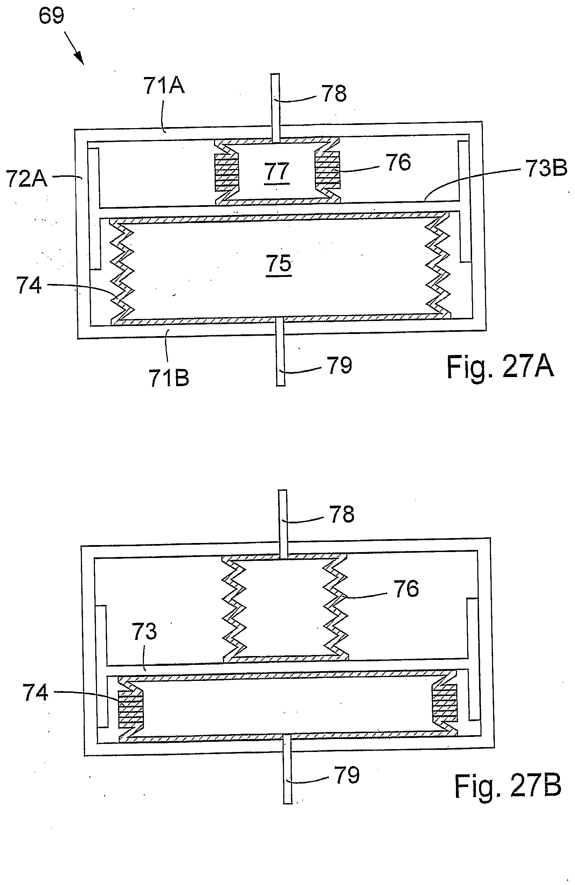

FIGURES 27A and 27B show a hydraulic reverse servo 69 suited for use in the embodiment of FIGURES 21 - 26. The reverse servo 69 includes a rectangular housing 70 with two parallel long sidewalls 71A and 71B and two parallel short sidewalls 72A and 72B. A movable wall 73 parallel with the long sidewalls 71A, 71B slides on the short sidewalls 72A, 72B between the long sidewalls 71A, 71B. A relatively large, substantially cylindrical bellows reservoir 74 defining a chamber 75 extends between and is joined to the movable intermediate wall 73 and the long sidewall 71B, and a relatively small, substantially cylindrical bellows reservoir 76 defining a chamber 77, which is substantially smaller than the chamber 75 of the large reservoir 74, extends between and is joined to the movable wall 73 and the long sidewall 71A. The small bellows reservoir 76 has a fluid supply pipe 78 for connection to the compressible reservoir 67 through the conduit 68A and the large bellows reservoir 74 has a fluid supply pipe 79 for connection to the bellows 59, 65A, 65B, 62A, 62B through the conduits 68B - 68D.

Referring to FIGURE 27A, when the patient compresses the reservoir 67, a small amount of hydraulic fluid is conducted from the reservoir 67 through the supply pipe 78 into the chamber 77 of the small bellows reservoir 76, so that the small bellows reservoir 76 expands and pushes the movable intermediate wall 73 towards the long sidewall 71B. As a result, the large bellows reservoir 74 is contracted by the intermediate wall 73 against the long sidewall 71B, whereby a large amount of hydraulic fluid is forced out of the chamber 75 of the large bellows reservoir 74 through the supply pipe 79 and further through the conduits 68B - 68D into the bellows 59, 65A, 65B, 62A, 62B of the pump 52, see FIGURE 27B.

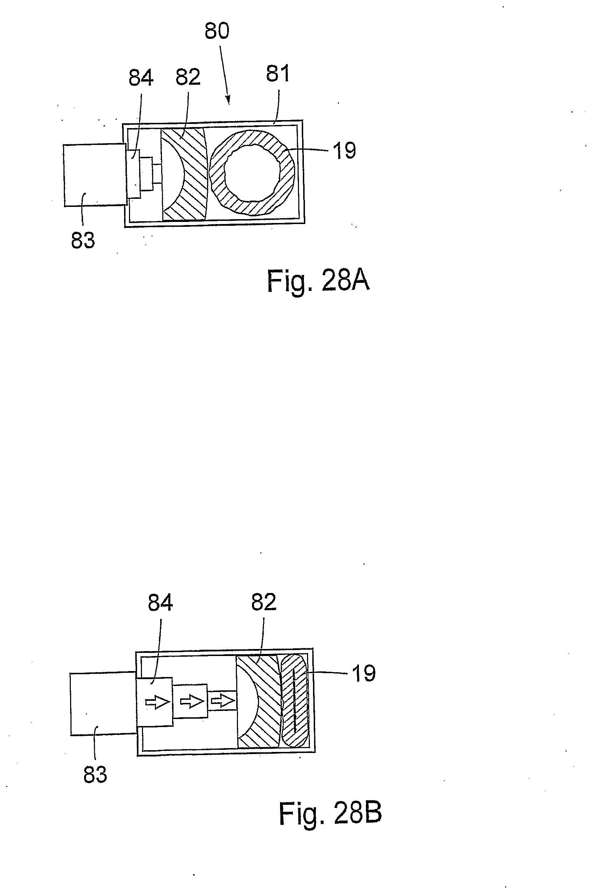

FIGURES 28A and 28B show a mechanically operated constriction device 80, which may be implemented in the embodiments according to FIGURES 1-10C and 15A, 15B. The constriction device 80 includes an open ended tubular housing 81 applied on a selected portion of a patient's intestine 19, and a constriction element 82, which is radially movable in the tubular housing 81 towards and away from the intestine 19 between a released position, see FIGURE 28A, and a constricted position, see FIGURE 28B, in which the constriction element 82 constricts the selected portion of the intestines 19. Mechanical operation means for mechanically operating the constriction element 82 includes an electric motor 83 attached to the housing 81 and a telescopic device 84, which is driven by the motor 83 and operatively connected to the constriction element 82. When the motor 83 is powered, it expands the telescopic device 30 so that the constriction element 82 constricts the intestine 19 and closes the passageway of the intestines 4, see FIGURE 28B. When the constriction element 82 is to release the intestine 19, the electric motor 83 is reversed so that the telescopic device 84 retracts the constriction element 82 to the position shown in FIGURE 28A, whereby the intestine 19 is released and the passageway of the intestines 4 is open.

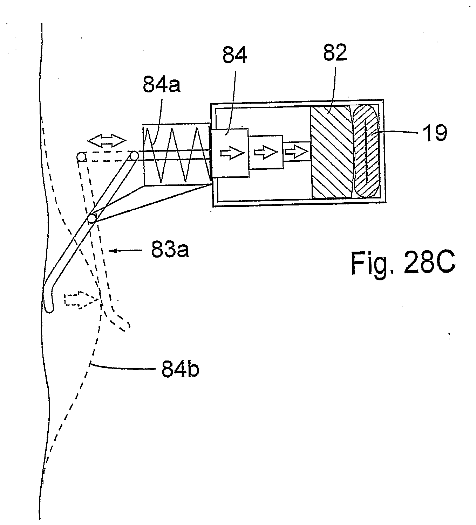

Alternatively, the mechanical operation means may include a subcutaneously implanted actuator operatively connected to clamping element 82, wherein the actuator is manually operated by the patient, as shown in FIGURE 28C. Thus, the motor 83 is replaced by a spring 84a acting to keep the telescopic device 84 expanded to force the clamping element 82 against the intestine 19. The actuator includes a lever mechanism 83a that is operatively connected to the telescopic device 84. The patient may push through the skin the lever mechanism 83a to pull the telescopic device 84 against the action of the spring 84a to the retracted position of the telescopic device 84, as indicated in phantom lines. When the patient releases the lever mechanism 83a, the spring 84a expands the telescopic device 84, whereby clamping element 82 is forced back against the intestine 19.

The mechanical operation means, as described above in connection with FIGURES 28A, 28B and 28C, may also be implemented in the embodiments according to FIGURES 1-10C and 15A-18.

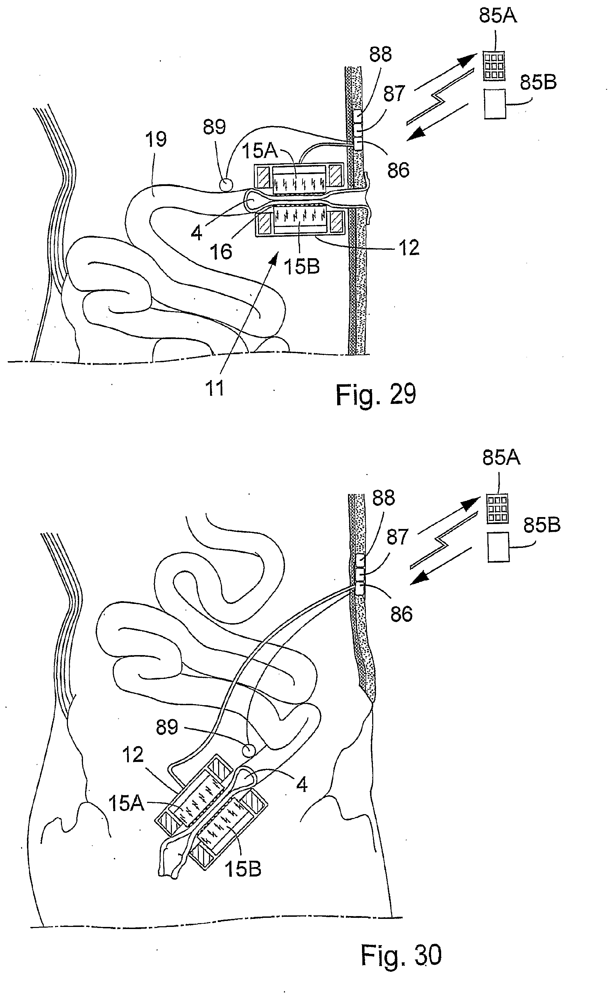

FIGURE 29 illustrates the pump 11 of the embodiment of FIGURES 6A and 6B applied on the intestines of a stoma patient, wherein the constriction elements 15A, 15B of the constriction device 12 constrict the intestines 19 and the electrodes 16 are energized to close the passageway of the intestines 4. A control device includes an external control unit in the form of a wireless remote control 85A, and an implanted internal control unit 86, which may include a microprocessor, for controlling the pump 11. The remote control 85A is operable by the patient to control the internal control unit 86 to switch on and off the pump 11. A separate wireless energy transmitter 85B, which alternatively may be integrated with the remote control 85A, is adapted to transmit wireless energy from outside the patient's body to an implanted energy-transforming device 87 that transforms the transmitted wireless energy into electric energy. An implanted rechargeable battery 88 for powering the pump 11 and for energizing the electrodes 16 stores the electric energy produced by the energy-transforming device 87. The control unit 86, energy-transforming device 87 and battery 88 are implanted as a package in the patient's fat layer between the skin and the abdominal wall. The pump 11 may also be directly powered with the electric energy, as the energy-transforming device 87 transforms the wireless energy transmitted by the wireless energy transmitter 85B into the electric energy. The wireless energy may comprise electromagnetic waves emitted by a coil of the energy transmitter 85B, wherein a corresponding coil of the energy-transforming device 87 transforms the electromagnetic waves into a current.

An implanted sensor 89 senses a physical parameter of the patient, such as the volume of the intestinal contents in the selected portion of the intestines or the distension of or the pressure in the intestines 19. The remote control 85A is adapted to produce an indication, such as a sound signal or displayed information, in response to the sensor sensing a value of the physical parameter exceeding a threshold value, when the pump 11 is not in operation. This indication should pay attention to the patient when it is time to defecate.

FIGURE 30 illustrates the pump 11 of the embodiment of FIGURES 6A and 6B applied on a colostomy patient's small intestines 19 surgically connected to the patient's anus.

## Claims

1. An apparatus for treating a patient having a disorder related to the passageway of the patient's intestines, **characterized in that** the apparatus comprises an electric pump (1) for implantation in the patient, said electric pump being operable on at least one selected portion (2) of intestinal tissue of the intestines (3) to pump intestinal contents through the passageway of the intestines, wherein said electric pump includes a constriction device (12) adapted to alternately constrict and release the selected portion to at least substantially reduce the volume of the passageway of the intestines along the selected portion and release the selected portion to increase the volume of the passageway of the intestines along the selected portion, such that intestinal contents is displaced through the passageway of the intestines (4).

2. The apparatus according to claim 1, said apparatus further comprising an implantable sensor (89) adapted to directly or indirectly sense a physical parameter of the patient or a functional parameter of the apparatus.

3. The apparatus according to claim 2, further comprising a control device (9) adapted produce an indication response to the sensor sensing a value exceeding a threshold value.

4. The apparatus according to any one of claims 2 and 3, wherein the physical parameter is at least one of the volume of the intestinal contents in the selected portion of the intestines, the distension of the intestinal wall, or the pressure in the selected portion of the intestines.

5. The apparatus according to any one of claims 3 and 4, wherein said indication is at least one of an alarm, a sound signal or displayed information.

6. The apparatus according to any one of claims 3-5, wherein said control device is further adapted to allow the user to control said electric pump to operate said constriction device to alternately constrict and release the selected portion, such that intestinal contents are moved through the passageway of the intestines.

7. The apparatus according to claim 6, wherein said control device comprises at least one of; a wireless remote control (85A) and a manually operable switch, said switch being adapted for subcutaneous implantation in the patient, for starting and stopping said electric pump, wherein said control device is operable by the patient.

8. The apparatus according to any one of claims 1-7, wherein the patient's intestines are surgically modified ending in at least one of; a natural or an artificial stoma and the patient's anus, and wherein said electric pump is adapted to pump intestinal contents out from the patient's body through the stoma or anus.

9. The apparatus according to anyone of claims 1-8, further comprising an engagement device attached to said electric pump and adapted to engage said electric pump with tissue related to the patient's abdominal cavity.

10. The apparatus according to any one of claims 1-8, wherein said constriction device is adapted to constrict the selected portion against a tissue of the patient's body.

11. The apparatus according to any one of claims 1-9, wherein said constriction device is adapted to constrict the selected portion against a tissue of the patient's body.

12. The apparatus according to any one of claims 1-11, further comprising a stimulation device for electrically stimulating muscle or neural tissue of the selected portion of the intestines to cause at least partial contraction of the selected portion, wherein said stimulation device comprises at least one electrode adapted to stimulate muscle or neural tissue of the selected portion of the intestinal tissue with electric pulses, further comprising a control device for controlling said stimulation device.

13. The apparatus according to claim 12, wherein said stimulation device comprises a plurality of electrodes (16) separate from or integrated with said constriction device, and said control device is adapted to control said stimulation device to variably energize said electrodes along the selected portion to cause partial contractions of the selected portion that over time change their positions on the selected portion.

14. The apparatus according to claim 13, wherein said control device is adapted to control said stimulation device to energize said electrodes, such that a number or groups of said electrodes are progressively energized in a direction upstream or downstream of the intestines.

15. The apparatus according to claim 13, wherein said control device is adapted to control said stimulation device to energize said electrodes, such that said electrodes are energized one at a time in sequence or groups of said electrodes are sequentially energized, either randomly or in accordance with a predetermined pattern.

16. The apparatus according to any one of claims 1-15, wherein said constriction device comprises at least one constriction element for constricting and releasing the selected portion and said control device is adapted to control said constriction element to alternately constrict and release the selected portion.

## Patentansprüche

1. Vorrichtung zum Behandeln eines Patienten mit einer Störung in Bezug auf den Darmdurchgang des Patienten, **dadurch gekennzeichnet, dass** die Vorrichtung eine elektrische Pumpe (1) zur Implantation in den Patienten umfasst, wobei die elektrische Pumpe an mindestens einem ausgewählten Abschnitt (2) des Darmgewebes des Darms (3) betreibbar ist, um Darminhalte durch den Darmdurchgang zu pumpen, wobei die elektrische Pumpe eine Konstriktionsvorrichtung (12) einschließt, die dazu ausgelegt ist, den ausgewählten Abschnitt alternierend zusammenzuziehen und loszulassen, um das Volumen des Darmdurchgangs entlang des ausgewählten Abschnitts zumindest wesentlich zu reduzieren und den ausgewählten Abschnitt loszulassen, um das Volumen des Darmdurchgangs entlang des ausgewählten Abschnitts zu erhöhen, so dass der Darminhalt durch den Darmdurchgang (4) geschoben wird.

2. Vorrichtung nach Anspruch 1, wobei die Vorrichtung ferner einen implantierbaren Sensor (89) umfasst, der dazu ausgelegt ist, einen physischen Parameter des Patienten oder einen funktionellen Parameter der Vorrichtung direkt oder indirekt abzufühlen.

3. Vorrichtung nach Anspruch 2, die ferner eine Steuervorrichtung (9) umfasst, die dazu ausgelegt ist, eine Angabe in Reaktion darauf zu produzieren, dass der Sensor einen Wert erfasst, der einen Schwellenwert überschreitet.

4. Vorrichtung nach einem der Ansprüche 2 und 3, wobei der physische Parameter mindestens eines von dem Volumen des Darminhalts in dem ausgewählten Abschnitt des Darms, der Distension der Darmwand oder dem Druck in dem ausgewählten Abschnitt des Darms ist.

5. Vorrichtung nach einem der Ansprüche 3 und 4, wobei die Angabe mindestens eines von einem Alarm, einem Tonsignal oder angezeigten Informationen ist.

6. Vorrichtung nach einem der Ansprüche 3-5, wobei die Steuervorrichtung ferner dazu ausgelegt ist, es dem Benutzer zu ermöglichen, die elektrische Pumpe zu steuern, um die Konstriktionsvorrichtung zu betreiben, um alternierend den ausgewählten Abschnitt zusammenzuziehen und loszulassen, so dass Darminhalte durch den Darmdurchgang bewegt werden.

7. Vorrichtung nach Anspruch 6, wobei die Steuervorrichtung mindestens eines von einer drahtlosen Fernbedienung (85A) und einem manuell bedienbaren Schalter umfasst, wobei der Schalter für eine subkutane Implantation in den Patienten ausgelegt ist, um die elektrische Pumpe zu starten und zu stoppen, wobei die Steuervorrichtung durch den Patienten bedienbar ist.

8. Vorrichtung nach einem der Ansprüche 1-7, wobei der Darm des Patienten chirurgisch modifiziert ist und in mindestens einem von einem natürlichen oder einem künstlichen Stoma und dem Anus des Patienten endet, und wobei die elektrische Pumpe dazu ausgelegt ist, Darminhalte aus dem Körper des Patienten durch das Stoma oder den Anus heraus zu pumpen.

9. Vorrichtung nach einem der Ansprüche 1-8, die ferner eine Eingriffsvorrichtung umfasst, die an der elektrischen Pumpe angebracht ist und dazu ausgelegt ist, die elektrische Pumpe mit Gewebe in Eingriff zu bringen, das mit der Bauchhöhle des Patienten in Beziehung steht.

10. Vorrichtung nach einem der Ansprüche 1-8, wobei die Konstriktionsvorrichtung dazu ausgelegt ist, den ausgewählten Abschnitt gegen ein Gewebe des Körpers des Patienten zusammenzuziehen.

11. Vorrichtung nach einem der Ansprüche 1-9, wobei die Konstriktionsvorrichtung dazu ausgelegt ist, den ausgewählten Abschnitt gegen ein Gewebe des Körpers des Patienten zusammenzuziehen.

12. Vorrichtung nach einem der Ansprüche 1-11, ferner umfassend eine Stimulationsvorrichtung zum elektrischen Stimulieren von Muskel- oder Nervengewebe des ausgewählten Abschnitts des Darms, um eine zumindest teilweise Kontraktion des ausgewählten Abschnitts zu bewirken, wobei die Stimulationsvorrichtung mindestens eine Elektrode umfasst, die dazu ausgelegt ist, Muskel- oder Nervengewebe des ausgewählten Abschnitts des Darmgewebes mit elektrischen Impulsen zu stimulieren, ferner umfassend eine Steuervorrichtung zum Steuern der Stimulationsvorrichtung.

13. Vorrichtung nach Anspruch 12, wobei die Stimulationsvorrichtung eine Vielzahl von Elektroden (16) umfasst, die separat von der Konstriktionsvorrichtung vorliegen oder in diese integriert sind, und die Steuervorrichtung dazu ausgelegt ist, die Stimulationsvorrichtung zu steuern, um die Elektroden entlang des ausgewählten Abschnitts variabel mit Energie zu versorgen, um partielle Kontraktionen des ausgewählten Abschnitts zu bewirken, die ihre Positionen auf dem ausgewählten Abschnitt im Zeitverlauf ändern.

14. Vorrichtung nach Anspruch 13, wobei die Steuervorrichtung dazu ausgelegt ist, die Stimulationsvorrichtung zu steuern, um die Elektroden mit Energie zu versorgen, so dass eine Anzahl oder Gruppen der Elektroden progressiv in einer Richtung stromaufwärts oder stromabwärts des Darms mit Energie versorgt werden.

15. Vorrichtung nach Anspruch 13, wobei die Steuervorrichtung dazu ausgelegt ist, die Stimulationsvorrichtung zu steuern, um die Elektroden mit Energie zu versorgen, so dass die Elektroden einzeln in Folge mit Energie versorgt werden oder Gruppen der Elektroden nacheinander entweder zufällig oder gemäß einem vorbestimmten Muster mit Energie versorgt werden.

16. Vorrichtung nach einem der Ansprüche 1-15, wobei die Konstriktionsvorrichtung mindestens ein Konstriktionselement zum Zusammenziehen und Loslassen des ausgewählten Abschnitts umfasst, und die Steuervorrichtung dazu ausgelegt ist, das Konstriktionselement zu steuern, um den ausgewählten Abschnitt alternierend zusammenzuziehen und loszulassen.

## Revendications

1. Appareil pour traiter un patient souffrant d'un trouble lié au passage des intestins du patient, **caractérisé en ce que** l'appareil comprend une pompe électrique (1) destinée à être implantée dans le patient, ladite pompe électrique pouvant fonctionner sur au moins une partie sélectionnée (2) du tissu intestinal des intestins (3) pour pomper le contenu intestinal à travers le passage des intestins, ladite pompe électrique comprenant un dispositif de constriction (12) conçu pour réaliser une constriction et libérer alternativement la partie sélectionnée afin de réduire au moins sensiblement le volume du passage des intestins le long de la partie sélectionnée et libérer la partie sélectionnée afin d'augmenter le volume du passage des intestins le long de la partie sélectionnée, de sorte que le contenu intestinal soit déplacé à travers le passage des intestins (4).

2. Appareil selon la revendication 1, ledit appareil comprenant en outre un capteur implantable (89) adapté pour détecter directement ou indirectement un paramètre physique du patient ou un paramètre fonctionnel de l'appareil.

3. Appareil selon la revendication 2, comprenant en outre un dispositif de commande (9) adapté pour produire une réponse d'indication au capteur détectant une valeur dépassant une valeur de seuil.

4. Appareil selon l'une quelconque des revendications 2 et 3, le paramètre physique étant au moins l'un du volume du contenu intestinal dans la partie sélectionnée des intestins, de la distension de la paroi intestinale ou de la pression dans la partie sélectionnée des intestins.

5. Appareil selon l'une quelconque des revendications 3 et 4, ladite indication étant au moins l'une d'une alarme, d'un signal sonore ou d'une information affichée.

6. Appareil selon l'une quelconque des revendications 3 à 5, ledit dispositif de commande étant en outre adapté pour permettre à l'utilisateur de commander ladite pompe électrique pour faire fonctionner ledit dispositif de constriction afin de réaliser une constriction et de libérer alternativement la partie sélectionnée, de sorte que le contenu intestinal soit déplacé à travers le passage des intestins.

7. Appareil selon la revendication 6, ledit dispositif de commande comprenant au moins l'un parmi : une télécommande sans fil (85A) et un commutateur actionnable manuellement, ledit commutateur étant adapté pour une implantation sous-cutanée dans le patient, pour démarrer et arrêter ladite pompe électrique, ledit dispositif de commande pouvant être actionné par le patient.

8. Appareil selon l'une quelconque des revendications 1 à 7, les intestins du patient étant modifiés chirurgicalement et se terminant par au moins l'un parmi : une stomie naturelle ou artificielle et l'anus du patient, et ladite pompe électrique étant adaptée pour pomper le contenu intestinal hors du corps du patient à travers la stomie ou l'anus.

9. Appareil selon l'une quelconque des revendications 1 à 8, comprenant en outre un dispositif d'engagement fixé à ladite pompe électrique et adapté pour engager ladite pompe électrique avec un tissu lié à la cavité abdominale du patient.

10. Appareil selon l'une quelconque des revendications 1 à 8, ledit dispositif de constriction étant adapté pour réaliser une constriction la partie sélectionnée contre un tissu du corps du patient.

11. Appareil selon l'une quelconque des revendications 1 à 9, ledit dispositif de constriction étant adapté pour réaliser une constriction la partie sélectionnée contre un tissu du corps du patient.

12. Appareil selon l'une quelconque des revendications 1 à 11, comprenant en outre un dispositif de stimulation pour stimuler électriquement le tissu musculaire ou neural de la partie sélectionnée des intestins pour provoquer une contraction au moins partielle de la partie sélectionnée, ledit dispositif de stimulation comprenant au moins une électrode adaptée pour stimuler le tissu musculaire ou neural de la partie sélectionnée du tissu intestinal avec des impulsions électriques, comprenant en outre un dispositif de commande pour commander ledit dispositif de stimulation.

13. Appareil selon la revendication 12, ledit dispositif de stimulation comprenant une pluralité d'électrodes (16) séparées dudit dispositif de constriction ou intégrées à celui-ci, et ledit dispositif de commande étant adapté pour commander ledit dispositif de stimulation pour exciter de manière variable lesdites électrodes le long de la partie sélectionnée pour provoquer des contractions partielles de la partie sélectionnée qui changent au fil du temps leurs positions sur la partie sélectionnée.

14. Appareil selon la revendication 13, ledit dispositif de commande étant adapté pour commander ledit dispositif de stimulation pour exciter lesdites électrodes, de sorte qu'un certain nombre ou des groupes desdites électrodes sont progressivement excités dans une direction en amont ou en aval des intestins.

15. Appareil selon la revendication 13, ledit dispositif de commande étant adapté pour commander ledit dispositif de stimulation pour exciter lesdites électrodes, de telle sorte que lesdites électrodes soient excitées une à une en séquence ou que des groupes desdites électrodes soient excités séquentiellement, soit de façon aléatoire, soit selon un motif prédéterminé.

16. Appareil selon l'une quelconque des revendications 1 à 15, ledit dispositif de constriction comprenant au moins un élément de constriction pour réaliser une constriction et libérer la partie sélectionnée, et ledit dispositif de commande étant adapté pour commander ledit élément de constriction pour réaliser une constriction et libérer alternativement la partie sélectionnée.
